# EUROPEAN PATENT APPLICATION

(11) **EP 0 550 296 A2**
(43) Date of publication of application: **07.07.1993**
(21) Application number: 92403199.0
(22) Date of filing: 27.11.1992
(51) Int. Cl.: C12N 15/18, C07K 13/00, C12P 21/02, C12N 5/10, A61K 37/36

(54) **Vascular endothelial cells growth factor**

(30) Priority: 28.11.1991 JP 337999/91
(71) Applicant: TERUMO KABUSHIKI KAISHA, Tokyo (JP)
(72) Inventor: Sudo, Tadashi, Ashigarakami-gun, Kanagawa-ken (JP); Harada, Kazumichi, Ashigarakami-gun, Kanagawa-ken (JP); Hirahara, Ichiro, Ashigarakami-gun, Kanagawa-ken (JP); Adachi, Masami, Ashigarakami-gun, Kanagawa-ken (JP)
(74) Representative: Gillard, Marie-Louise

(57) **Abstract**

A novel protein of human origin produced by a human ovarian tumor established cell line HUOCA-II or HUOCA-III, which has a molecular weight, when determined by SDS-polyacrylamide gel electrophoresis, of from 72,000 to 80,000 daltons under a non-reducing condition or from 79,000 to 85,000 daltons under a reducing condition, which contains an amino acid sequence represented by the Sequence ID No. 4 deduced from the DNA sequence represented by the Sequence ID No. 5, and which enhances growth of vascular endothelial cells but does not activate growth of smooth muscle cells, fibroblasts and hepatocytes and also does not enhance or inhibit growth of HeLa cells. This invention also provides a process for the production of the protein.

SEQUENCE LISTING

(1) GENERAL INFORMATION:

(i) APPLICANT:
(A) NAME: TERUMO KABUSHIKI KAISHA
(B) STREET: 44-1, Hatagaya 2-chome, Shibuya-ku
(C) CITY: TOKYO
(E) COUNTRY: JAPAN
(F) POSTAL CODE (ZIP): 151

(ii) TITLE OF INVENTION: Novel protein of human origin and its
production process

(iii) NUMBER OF SEQUENCES: 7
(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 92 403 199.0
(vi) PRIOR APPLICATION DATA:
(A) APPLICATION NUMBER: JP 3-337999
(B) FILING DATE: 28-NOV-1991

(2) INFORMATION FOR SEQ ID NO: 1:

(i) SEQUENCE CHARACTERISTICS:
(A) LENGTH: 7 amino acids
(B) TYPE: amino acid
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Homo sapiens
(G) CELL TYPE: Ovarian
(H) CELL LINE: HUOCA II / HUOCA III

(xi) SEQUENCE DESCRIPTION: SEQ ID NO: 1:

Arg Asn Thr Ile His Glu Phe
1               5

(2) INFORMATION FOR SEQ ID NO: 2:

(i) SEQUENCE CHARACTERISTICS:
(A) LENGTH: 10 amino acids
(B) TYPE: amino acid
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Homo sapiens
(G) CELL TYPE: Ovarian
(H) CELL LINE: HUOCA II / HUOCA III

(xi) SEQUENCE DESCRIPTION: SEQ ID NO: 2:

Glu Phe Gly His Glu Phe Asp Leu Tyr Glu
1               5                   10

(2) INFORMATION FOR SEQ ID NO: 3:

(i) SEQUENCE CHARACTERISTICS:
(A) LENGTH: 16 amino acids
(B) TYPE: amino acid
(D) TOPOLOGY: linear

(ii) MOLECULE TYPE: peptide

(iii) HYPOTHETICAL: NO

(v) FRAGMENT TYPE: C-terminal

(vi) ORIGINAL SOURCE:
(A) ORGANISM: Homo sapiens
(G) CELL TYPE: Ovarian
(H) CELL LINE: HUOCA II / HUOCA III

(ix) FEATURE:
(A) NAME/KEY: Modified-site
(B) LOCATION: 3
(D) OTHER INFORMATION: /label= Xaa
/note= "unidentified amino acid residue"

(ix) FEATURE:
(A) NAME/KEY: Modified-site
(B) LOCATION: 10
(D) OTHER INFORMATION: /label= Xaa
/note= "unidentified amino acid residue"

(xi) SEQUENCE DESCRIPTION: SEQ ID NO: 3:

Glu Ser Xaa Val Leu Thr Ala Arg Gln Xaa Phe Pro Ser Arg Asp Leu
1               5                   10                  15

(2) INFORMATION FOR SEQ ID NO: 4:

(i) SEQUENCE CHARACTERISTICS:
(A) LENGTH: 728 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Homo sapiens
(G) CELL TYPE: ovarian
(H) CELL LINE: HUOCA II / HUOCA III

(xi) SEQUENCE DESCRIPTION: SEQ ID NO: 4:

Met Trp Val Thr Lys Leu Leu Pro Ala Leu Leu Leu Gln His Val Leu
1               5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr
        35                  40                  45

Thr Leu Ile Lys Ile Asp Pro Ala Leu Lys Ile Lys Thr Lys Lys Val
    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys
        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Phe Leu Pro Ser Ser Tyr Arg Gly Lys Asp Leu Gln Glu Asn Tyr
                165                 170                 175
Cys Arg Asn Pro Arg Gly Glu Glu Gly Gly Pro Trp Cys Phe Thr Ser
            180                 185                 190
Asn Pro Glu Val Arg Tyr Glu Val Cys Asp Ile Pro Gln Cys Ser Glu
        195                 200                 205
Val Glu Cys Met Thr Cys Asn Gly Glu Ser Tyr Arg Gly Leu Met Asp
    210                 215                 220
His Thr Glu Ser Gly Lys Ile Cys Gln Arg Trp Asp His Gln Thr Pro
225                 230                 235                 240
His Arg His Lys Phe Leu Pro Glu Arg Tyr Pro Asp Lys Gly Phe Asp
                245                 250                 255
Asp Asn Tyr Cys Arg Asn Pro Asp Gly Gln Pro Arg Pro Trp Cys Tyr
            260                 265                 270
Thr Leu Asp Pro His Thr Arg Trp Glu Tyr Cys Ala Ile Lys Thr Cys
        275                 280                 285
Ala Asp Asn Thr Met Asn Asp Thr Asp Val Pro Leu Glu Thr Thr Glu
    290                 295                 300
Cys Ile Gln Gly Gln Gly Glu Gly Tyr Arg Gly Thr Val Asn Thr Ile
305                 310                 315                 320
Trp Asn Gly Ile Pro Cys Gln Arg Trp Asp Ser Gln Tyr Pro His Glu
                325                 330                 335
His Asp Met Thr Pro Glu Asn Phe Lys Cys Lys Asp Leu Arg Glu Asn
            340                 345                 350

Tyr Cys Arg Asn Pro Asp Gly Ser Glu Ser Pro Trp Cys Phe Thr Thr
        355                 360                 365
Asp Pro Asn Ile Arg Val Gly Tyr Cys Ser Gln Ile Pro Asn Cys Asp
    370                 375                 380
Met Ser His Gly Gln Asp Cys Tyr Arg Gly Asn Gly Lys Asn Tyr Met
385                 390                 395                 400
Gly Asn Leu Ser Gln Thr Arg Ser Gly Leu Thr Cys Ser Met Trp Asp
                405                 410                 415
Lys Asn Met Glu Asp Leu His Arg His Ile Phe Trp Glu Pro Asp Ala
            420                 425                 430
Ser Lys Leu Asn Glu Asn Tyr Cys Arg Asn Pro Asp Asp Asp Ala His
        435                 440                 445
Gly Pro Trp Cys Tyr Thr Gly Asn Pro Leu Ile Pro Trp Asp Tyr Cys
    450                 455                 460
Pro Ile Ser Arg Cys Glu Gly Asp Thr Thr Pro Thr Ile Val Asn Leu
465                 470                 475                 480
Asp His Pro Val Ile Ser Cys Ala Lys Thr Lys Gln Leu Arg Val Val
                485                 490                 495
Asn Gly Ile Pro Thr Arg Thr Asn Ile Gly Trp Met Val Ser Leu Arg
            500                 505                 510
Tyr Arg Asn Lys His Ile Cys Gly Gly Ser Leu Ile Lys Glu Ser Trp
        515                 520                 525
Val Leu Thr Ala Arg Gln Cys Phe Pro Ser Arg Asp Leu Lys Asp Tyr
    530                 535                 540
Glu Ala Trp Leu Gly Ile His Asp Val His Gly Arg Gly Asp Glu Lys
545                 550                 555                 560
Cys Lys Gln Val Leu Asn Val Ser Gln Leu Val Tyr Gly Pro Glu Gly
                565                 570                 575
Ser Asp Leu Val Leu Met Lys Leu Ala Arg Pro Ala Val Leu Asp Asp
            580                 585                 590
Phe Val Ser Thr Ile Asp Leu Pro Asn Tyr Gly Cys Thr Ile Pro Glu
        595                 600                 605
Lys Thr Ser Cys Ser Val Tyr Gly Trp Gly Tyr Thr Gly Leu Ile Asn
    610                 615                 620
Tyr Asp Gly Leu Leu Arg Val Ala His Leu Tyr Ile Met Gly Asn Glu
625                 630                 635                 640
Lys Cys Ser Gln His His Arg Gly Lys Val Thr Leu Asn Glu Ser Glu
                645                 650                 655

Ile Cys Ala Gly Ala Glu Lys Ile Gly Ser Gly Pro Cys Glu Gly Asp
            660                 665                 670
Tyr Gly Gly Pro Leu Val Cys Glu Gln His Lys Met Arg Met Val Leu
        675                 680                 685
Gly Val Ile Val Pro Gly Arg Gly Cys Ala Ile Pro Asn Arg Pro Gly
    690                 695                 700
Ile Phe Val Arg Val Ala Tyr Tyr Ala Lys Trp Ile His Lys Ile Ile
705                 710                 715                 720
Leu Thr Tyr Lys Val Pro Gln Ser
                725

(2) INFORMATION FOR SEQ ID NO: 5:

(i) SEQUENCE CHARACTERISTICS:
(A) LENGTH: 2187 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: YES

(iii) ANTI-SENSE: NO

(xi) SEQUENCE DESCRIPTION: SEQ ID NO: 5:

ATGTGGGTGA CCAAACTCCT GCCAGCCCTG CTGCTGCAGC ATGTCCTCCT GCATCTCCTC 60
CTGCTCCCCA TCGCCATCCC CTATGCAGAG GGACAAAGGA AAAGAAGAAA TACAATTCAT 120
GAATTCAAAA AATCAGCAAA GACTACCCTA ATCAAAATAG ATCCAGCACT GAAGATAAAA 180
ACCAAAAAAG TGAATACTGC AGACCAATGT GCTAATAGAT GTACTAGGAA TAAAGGACTT 240
CCATTCACTT GCAAGGCTTT TGTTTTTGAT AAAGCAAGAA AACAATGCCT CTGGTTCCCC 300
TTCAATAGCA TGTCAAGTGG AGTGAAAAAA GAATTTGGCC ATGAATTTGA CCTCTATGAA 360
AACAAAGACT ACATTAGAAA CTGCATCATT GGTAAAGGAC GCAGCTACAA GGGAACAGTA 420
TCTATCACTA AGAGTGGCAT CAAATGTCAG CCCTGGAGTT CCATGATACC ACACGAACAC 480
AGCTTTTTGC CTTCGAGCTA TCGGGGTAAA GACCTACAGG AAAACTACTG TCGAAATCCT 540
CGAGGGGAAG AAGGGGGACC CTGGTGTTTC ACAAGCAATC CAGAGGTACG CTACGAAGTC 600
TGTGACATTC CTCAGTGTTC AGAAGTTGAA TGCATGACCT GCAATGGGGA GAGTTATCGA 660
GGTCTCATGG ATCATACAGA ATCAGGCAAG ATTTGTCAGC GCTGGGATCA TCAGACACCA 720
CACCGGCACA AATTCTTGCC TGAAAGATAT CCCGACAAGG GCTTTGATGA TAATTATTGC 780
CGCAATCCCG ATGGCCAGCC GAGGCCATGG TGCTATACTC TTGACCCTCA CACCCGCTGG 840
GAGTACTGTG CAATTAAAAC ATGCGCTGAC AATACTATGA ATGACACTGA TGTTCCTTTG 900
GAAACAACTG AATGCATCCA AGGTCAAGGA GAAGGCTACA GGGGCACTGT CAATACCATT 960
TGGAATGGAA TTCCATGTCA GCGTTGGGAT TCTCAGTATC CTCACGAGCA TGACATGACT 1020

CCTGAAAATT TCAAGTGCAA GGACCTACGA GAAAATTACT GCCGAAATCC AGATGGGTCT 1080
GAATCACCCT GGTGTTTTAC CACTGATCCA AACATCCGAG TTGGCTACTG CTCCCAAATT 1140
CCAAACTGTG ATATGTCACA TGGACAAGAT TGTTATCGTG GGAATGGCAA AAATTATATG 1200
GGCAACTTAT CCCAAACAAG ATCTGGACTA ACATGTTCAA TGTGGGACAA GAACATGGAA 1260
GACTTACATC GTCATATCTT CTGGGAACCA GATGCAAGTA AGCTGAATGA GAATTACTGC 1320
CGAAATCCAG ATGATGATGC TCATGGACCC TGGTGCTACA CGGGAAATCC ACTCATTCCT 1380
TGGGATTATT GCCCTATTTC TCGTTGTGAA GGTGATACCA CACCTACAAT AGTCAATTTA 1440
GACCATCCCG TAATATCTTG TGCCAAAACG AAACAATTGC GAGTTGTAAA TGGGATTCCA 1500
ACACGAACAA ACATAGGATG GATGGTTAGT TTGAGATACA GAAATAAACA TATCTGCGGA 1560
GGATCATTGA TAAAGGAGAG TTGGGTTCTT ACTGCACGAC AGTGTTTCCC TTCTCGAGAC 1620
TTGAAAGATT ATGAAGCTTG GCTTGGAATT CATGATGTCC ACGGAAGAGG AGATGAGAAA 1680
TGCAAACAGG TTCTCAATGT TTCCCAGCTG GTATATGGCC CTGAAGGATC AGATCTGGTT 1740
TTAATGAAGC TTGCCAGGCC TGCTGTCCTG GATGATTTTG TTAGTACGAT TGATTTACCT 1800
AATTATGGAT GCACAATTCC TGAAAAGACC AGTTGCAGTG TTTATGGCTG GGGCTACACT 1860
GGATTGATCA ACTATGATGG CCTATTACGA GTGGCACATC TCTATATAAT GGGAAATGAG 1920
AAATGCAGCC AGCATCATCG AGGGAAGGTG ACTCTGAATG AGTCTGAAAT ATGTGCTGGG 1980
GCTGAAAAGA TTGGATCAGG ACCATGTGAG GGGGATTATG GTGGCCCACT TGTTTGTGAG 2040
CAACATAAAA TGAGAATGGT TCTTGGTGTC ATTGTTCCTG GTCGTGGATG TGCCATTCCA 2100
AATCGTCCTG GTATTTTTGT CCGAGTAGCA TATTATGCAA AATGGATACA CAAAATTATT 2160
TTAACATATA AGGTACCACA GTCATAG 2187

(2) INFORMATION FOR SEQ ID NO: 6:

(i) SEQUENCE CHARACTERISTICS:
(A) LENGTH: 2576 base pairs
(B) TYPE: nucleic acid
(C) STRANDEDNESS: single
(D) TOPOLOGY: linear

(ii) MOLECULE TYPE: mRNA

(iii) HYPOTHETICAL: YES

(iii) ANTI-SENSE: NO

(ix) FEATURE:
(A) NAME/KEY: CDS
(B) LOCATION: join(102..2285, 2289..2294, 2298..2336, 2340
..2384, 2388..2480, 2484..2507, 2514..2522, 2526
..2570)

(xi) SEQUENCE DESCRIPTION: SEQ ID NO: 6:

GGGCUCAGAG CCGACUGGCU CUUUUAGGCA CUGACUCCGA ACAGGAUUCU UUCACCCAGG 60

CAUCUCCUCC AGAGGGAUCC GCCAGCCCGU CCAGCAGCAC C AUG UGG GUG ACC 113
Met Trp Val Thr
1

AAA CUC CUG CCA GCC CUG CUG CUG CAG CAU GUC CUC CUG CAU CUC CUC 161
Lys Leu Leu Pro Ala Leu Leu Leu Gln His Val Leu Leu His Leu Leu
5                   10                  15                  20

CUG CUC CCC AUC GCC AUC CCC UAU GCA GAG GGA CAA AGG AAA AGA AGA 209
Leu Leu Pro Ile Ala Ile Pro Tyr Ala Glu Gly Gln Arg Lys Arg Arg
                25                  30                  35

AAU ACA AUU CAU GAA UUC AAA AAA UCA GCA AAG ACU ACC CUA AUC AAA 257
Asn Thr Ile His Glu Phe Lys Lys Ser Ala Lys Thr Thr Leu Ile Lys
            40                  45                  50

AUA GAU CCA GCA CUG AAG AUA AAA ACC AAA AAA GUG AAU ACU GCA GAC 305
Ile Asp Pro Ala Leu Lys Ile Lys Thr Lys Lys Val Asn Thr Ala Asp
        55                  60                  65

CAA UGU GCU AAU AGA UGU ACU AGG AAU AAA GGA CUU CCA UUC ACU UGC 353
Gln Cys Ala Asn Arg Cys Thr Arg Asn Lys Gly Leu Pro Phe Thr Cys
    70                  75                  80

AAG GCU UUU GUU UUU GAU AAA GCA AGA AAA CAA UGC CUC UGG UUC CCC 401
Lys Ala Phe Val Phe Asp Lys Ala Arg Lys Gln Cys Leu Trp Phe Pro
85                  90                  95                  100

UUC AAU AGC AUG UCA AGU GGA GUG AAA AAA GAA UUU GGC CAU GAA UUU 449
Phe Asn Ser Met Ser Ser Gly Val Lys Lys Glu Phe Gly His Glu Phe
                105                 110                 115

GAC CUC UAU GAA AAC AAA GAC UAC AUU AGA AAC UGC AUC AUU GGU AAA 497
Asp Leu Tyr Glu Asn Lys Asp Tyr Ile Arg Asn Cys Ile Ile Gly Lys
            120                 125                 130

GGA CGC AGC UAC AAG GGA ACA GUA UCU AUC ACU AAG AGU GGC AUC AAA 545
Gly Arg Ser Tyr Lys Gly Thr Val Ser Ile Thr Lys Ser Gly Ile Lys
        135                 140                 145

UGU CAG CCC UGG AGU UCC AUG AUA CCA CAC GAA CAC AGC UUU UUG CCU 593
Cys Gln Pro Trp Ser Ser Met Ile Pro His Glu His Ser Phe Leu Pro
    150                 155                 160

UCG AGC UAU CGG GGU AAA GAC CUA CAG GAA AAC UAC UGU CGA AAU CCU 641
Ser Ser Tyr Arg Gly Lys Asp Leu Gln Glu Asn Tyr Cys Arg Asn Pro
165                 170                 175                 180

CGA GGG GAA GAA GGG GGA CCC UGG UGU UUC ACA AGC AAU CCA GAG GUA 689
Arg Gly Glu Glu Gly Gly Pro Trp Cys Phe Thr Ser Asn Pro Glu Val
                185                 190                 195

CGC UAC GAA GUC UGU GAC AUU CCU CAG UGU UCA GAA GUU GAA UGC AUG 737
Arg Tyr Glu Val Cys Asp Ile Pro Gln Cys Ser Glu Val Glu Cys Met
            200                 205                 210

ACC UGC AAU GGG GAG AGU UAU CGA GGU CUC AUG GAU CAU ACA GAA UCA 785
Thr Cys Asn Gly Glu Ser Tyr Arg Gly Leu Met Asp His Thr Glu Ser
        215                 220                 225

GGC AAG AUU UGU CAG CGC UGG GAU CAU CAG ACA CCA CAC CGG CAC AAA 833
Gly Lys Ile Cys Gln Arg Trp Asp His Gln Thr Pro His Arg His Lys
    230                 235                 240

UUC UUG CCU GAA AGA UAU CCC GAC AAG GGC UUU GAU GAU AAU UAU UGC 881
Phe Leu Pro Glu Arg Tyr Pro Asp Lys Gly Phe Asp Asp Asn Tyr Cys
245                 250                 255                 260

CGC AAU CCC GAU GGC CAG CCG AGG CCA UGG UGC UAU ACU CUU GAC CCU 929
Arg Asn Pro Asp Gly Gln Pro Arg Pro Trp Cys Tyr Thr Leu Asp Pro
                265                 270                 275

CAC ACC CGC UGG GAG UAC UGU GCA AUU AAA ACA UGC GCU GAC AAU ACU 977
His Thr Arg Trp Glu Tyr Cys Ala Ile Lys Thr Cys Ala Asp Asn Thr
            280                 285                 290

AUG AAU GAC ACU GAU GUU CCU UUG GAA ACA ACU GAA UGC AUC CAA GGU 1025
Met Asn Asp Thr Asp Val Pro Leu Glu Thr Thr Glu Cys Ile Gln Gly
        295                 300                 305

CAA GGA GAA GGC UAC AGG GGC ACU GUC AAU ACC AUU UGG AAU GGA AUU 1073
Gln Gly Glu Gly Tyr Arg Gly Thr Val Asn Thr Ile Trp Asn Gly Ile
    310                 315                 320

CCA UGU CAG CGU UGG GAU UCU CAG UAU CCU CAC GAG CAU GAC AUG ACU 1121
Pro Cys Gln Arg Trp Asp Ser Gln Tyr Pro His Glu His Asp Met Thr
325                 330                 335                 340

CCU GAA AAU UUC AAG UGC AAG GAC CUA CGA GAA AAU UAC UGC CGA AAU 1169
Pro Glu Asn Phe Lys Cys Lys Asp Leu Arg Glu Asn Tyr Cys Arg Asn
                345                 350                 355

CCA GAU GGG UCU GAA UCA CCC UGG UGU UUU ACC ACU GAU CCA AAC AUC 1217
Pro Asp Gly Ser Glu Ser Pro Trp Cys Phe Thr Thr Asp Pro Asn Ile
            360                 365                 370

CGA GUU GGC UAC UGC UCC CAA AUU CCA AAC UGU GAU AUG UCA CAU GGA 1265
Arg Val Gly Tyr Cys Ser Gln Ile Pro Asn Cys Asp Met Ser His Gly
        375                 380                 385

CAA GAU UGU UAU CGU GGG AAU GGC AAA AAU UAU AUG GGC AAC UUA UCC 1313
Gln Asp Cys Tyr Arg Gly Asn Gly Lys Asn Tyr Met Gly Asn Leu Ser
    390                 395                 400

CAA ACA AGA UCU GGA CUA ACA UGU UCA AUG UGG GAC AAG AAC AUG GAA 1361
Gln Thr Arg Ser Gly Leu Thr Cys Ser Met Trp Asp Lys Asn Met Glu
405                 410                 415                 420

GAC UUA CAU CGU CAU AUC UUC UGG GAA CCA GAU GCA AGU AAG CUG AAU 1409
Asp Leu His Arg His Ile Phe Trp Glu Pro Asp Ala Ser Lys Leu Asn
                425                 430                 435

GAG AAU UAC UGC CGA AAU CCA GAU GAU GAU GCU CAU GGA CCC UGG UGC 1457
Glu Asn Tyr Cys Arg Asn Pro Asp Asp Asp Ala His Gly Pro Trp Cys
            440                 445                 450

UAC ACG GGA AAU CCA CUC AUU CCU UGG GAU UAU UGC CCU AUU UCU CGU 1505
Tyr Thr Gly Asn Pro Leu Ile Pro Trp Asp Tyr Cys Pro Ile Ser Arg
        455                 460                 465

UGU GAA GGU GAU ACC ACA CCU ACA AUA GUC AAU UUA GAC CAU CCC GUA 1553
Cys Glu Gly Asp Thr Thr Pro Thr Ile Val Asn Leu Asp His Pro Val
    470                 475                 480

AUA UCU UGU GCC AAA ACG AAA CAA UUG CGA GUU GUA AAU GGG AUU CCA 1601
Ile Ser Cys Ala Lys Thr Lys Gln Leu Arg Val Val Asn Gly Ile Pro
485                 490                 495                 500

ACA CGA ACA AAC AUA GGA UGG AUG GUU AGU UUG AGA UAC AGA AAU AAA 1649
Thr Arg Thr Asn Ile Gly Trp Met Val Ser Leu Arg Tyr Arg Asn Lys
                505                 510                 515

CAU AUC UGC GGA GGA UCA UUG AUA AAG GAG AGU UGG GUU CUU ACU GCA 1697
His Ile Cys Gly Gly Ser Leu Ile Lys Glu Ser Trp Val Leu Thr Ala
            520                 525                 530

CGA CAG UGU UUC CCU UCU CGA GAC UUG AAA GAU UAU GAA GCU UGG CUU 1745
Arg Gln Cys Phe Pro Ser Arg Asp Leu Lys Asp Tyr Glu Ala Trp Leu
        535                 540                 545

GGA AUU CAU GAU GUC CAC GGA AGA GGA GAU GAG AAA UGC AAA CAG GUU 1793
Gly Ile His Asp Val His Gly Arg Gly Asp Glu Lys Cys Lys Gln Val
    550                 555                 560

CUC AAU GUU UCC CAG CUG GUA UAU GGC CCU GAA GGA UCA GAU CUG GUU 1841
Leu Asn Val Ser Gln Leu Val Tyr Gly Pro Glu Gly Ser Asp Leu Val
565                 570                 575                 580

UUA AUG AAG CUU GCC AGG CCU GCU GUC CUG GAU GAU UUU GUU AGU ACG 1889
Leu Met Lys Leu Ala Arg Pro Ala Val Leu Asp Asp Phe Val Ser Thr
                585                 590                 595

AUU GAU UUA CCU AAU UAU GGA UGC ACA AUU CCU GAA AAG ACC AGU UGC 1937
Ile Asp Leu Pro Asn Tyr Gly Cys Thr Ile Pro Glu Lys Thr Ser Cys
            600                 605                 610

AGU GUU UAU GGC UGG GGC UAC ACU GGA UUG AUC AAC UAU GAU GGC CUA 1985
Ser Val Tyr Gly Trp Gly Tyr Thr Gly Leu Ile Asn Tyr Asp Gly Leu
        615                 620                 625

UUA CGA GUG GCA CAU CUC UAU AUA AUG GGA AAU GAG AAA UGC AGC CAG 2033
Leu Arg Val Ala His Leu Tyr Ile Met Gly Asn Glu Lys Cys Ser Gln
    630                 635                 640

CAU CAU CGA GGG AAG GUG ACU CUG AAU GAG UCU GAA AUA UGU GCU GGG 2081
His His Arg Gly Lys Val Thr Leu Asn Glu Ser Glu Ile Cys Ala Gly
645                 650                 655                 660

GCU GAA AAG AUU GGA UCA GGA CCA UGU GAG GGG GAU UAU GGU GGC CCA 2129
Ala Glu Lys Ile Gly Ser Gly Pro Cys Glu Gly Asp Tyr Gly Gly Pro
                665                 670                 675

CUU GUU UGU GAG CAA CAU AAA AUG AGA AUG GUU CUU GGU GUC AUU GUU 2177
Leu Val Cys Glu Gln His Lys Met Arg Met Val Leu Gly Val Ile Val
            680                 685                 690

CCU GGU CGU GGA UGU GCC AUU CCA AAU CGU CCU GGU AUU UUU GUC CGA 2225
Pro Gly Arg Gly Cys Ala Ile Pro Asn Arg Pro Gly Ile Phe Val Arg
        695                 700                 705

GUA GCA UAU UAU GCA AAA UGG AUA CAC AAA AUU AUU UUA ACA UAU AAG 2273
Val Ala Tyr Tyr Ala Lys Trp Ile His Lys Ile Ile Leu Thr Tyr Lys
    710                 715                 720

GUA CCA CAG UCA UAG CUG AAG UAA GUG UGU CUG AAG CAC CCA CCA AUA 2321
Val Pro Gln Ser Leu Lys Val Cys Leu Lys His Pro Pro Ile
725                 730                 735

CAA CUG UCU UUU ACA UGA AGA UUU CAG AGA AUG UGG AAU UUA AAA UGU 2369
Gln Leu Ser Phe Thr Arg Phe Gln Arg Met Trp Asn Leu Lys Cys
    740                 745                 750

CAC UUA CAA CAA UCC UAA GAC AAC UAC UGG AGA GUC AUG UUU GUU GAA 2417
His Leu Gln Gln Ser Asp Asn Tyr Trp Arg Val Met Phe Val Glu
    755                 760                 765

AUU CUC AUU AAU GUU UAU GGG UGU UUU CUG UUG UUU UGU UUG UCA GUG 2465
Ile Leu Ile Asn Val Tyr Gly Cys Phe Leu Leu Phe Cys Leu Ser Val
    770                 775                 780

UUA UUU UGU CAA UGU UGA AGU GAA UUA AGG UAC AUG CAA GUG 2507
Leu Phe Cys Gln Cys Ser Glu Leu Arg Tyr Met Gln Val
785                 790                 795

UAAUAA CAU AUC UCC UGA AGA UAC UUG AAU GGA UUA AAA AAA CAC ACA 2555
His Ile Ser Arg Tyr Leu Asn Gly Leu Lys Lys His Thr
        800                 805                 810

GGU AUA UUU GCU GGA UGAUAA 2576
Gly Ile Phe Ala Gly
                815

(2) INFORMATION FOR SEQ ID NO: 7:

(i) SEQUENCE CHARACTERISTICS:
(A) LENGTH: 815 amino acids
(B) TYPE: amino acid
(D) TOPOLOGY: linear

(ii) MOLECULE TYPE: protein

(xi) SEQUENCE DESCRIPTION: SEQ ID NO: 7:

Met Trp Val Thr Lys Leu Leu Pro Ala Leu Leu Leu Gln His Val Leu
1               5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr
        35                  40                  45
Thr Leu Ile Lys Ile Asp Pro Ala Leu Lys Ile Lys Thr Lys Lys Val
    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys
        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Phe Leu Pro Ser Ser Tyr Arg Gly Lys Asp Leu Gln Glu Asn Tyr
                165                 170                 175
Cys Arg Asn Pro Arg Gly Glu Glu Gly Gly Pro Trp Cys Phe Thr Ser
            180                 185                 190
Asn Pro Glu Val Arg Tyr Glu Val Cys Asp Ile Pro Gln Cys Ser Glu
        195                 200                 205
Val Glu Cys Met Thr Cys Asn Gly Glu Ser Tyr Arg Gly Leu Met Asp
    210                 215                 220
His Thr Glu Ser Gly Lys Ile Cys Gln Arg Trp Asp His Gln Thr Pro
225                 230                 235                 240
His Arg His Lys Phe Leu Pro Glu Arg Tyr Pro Asp Lys Gly Phe Asp
                245                 250                 255
Asp Asn Tyr Cys Arg Asn Pro Asp Gly Gln Pro Arg Pro Trp Cys Tyr
            260                 265                 270
Thr Leu Asp Pro His Thr Arg Trp Glu Tyr Cys Ala Ile Lys Thr Cys
        275                 280                 285

Ala Asp Asn Thr Met Asn Asp Thr Asp Val Pro Leu Glu Thr Thr Glu
    290                 295                 300
Cys Ile Gln Gly Gln Gly Glu Gly Tyr Arg Gly Thr Val Asn Thr Ile
305                 310                 315                 320
Trp Asn Gly Ile Pro Cys Gln Arg Trp Asp Ser Gln Tyr Pro His Glu
                325                 330                 335
His Asp Met Thr Pro Glu Asn Phe Lys Cys Lys Asp Leu Arg Glu Asn
            340                 345                 350
Tyr Cys Arg Asn Pro Asp Gly Ser Glu Ser Pro Trp Cys Phe Thr Thr
        355                 360                 365
Asp Pro Asn Ile Arg Val Gly Tyr Cys Ser Gln Ile Pro Asn Cys Asp
    370                 375                 380
Met Ser His Gly Gln Asp Cys Tyr Arg Gly Asn Gly Lys Asn Tyr Met
385                 390                 395                 400
Gly Asn Leu Ser Gln Thr Arg Ser Gly Leu Thr Cys Ser Met Trp Asp
                405                 410                 415
Lys Asn Met Glu Asp Leu His Arg His Ile Phe Trp Glu Pro Asp Ala
            420                 425                 430
Ser Lys Leu Asn Glu Asn Tyr Cys Arg Asn Pro Asp Asp Asp Ala His
        435                 440                 445
Gly Pro Trp Cys Tyr Thr Gly Asn Pro Leu Ile Pro Trp Asp Tyr Cys
    450                 455                 460
Pro Ile Ser Arg Cys Glu Gly Asp Thr Thr Pro Thr Ile Val Asn Leu
465                 470                 475                 480
Asp His Pro Val Ile Ser Cys Ala Lys Thr Lys Gln Leu Arg Val Val
                485                 490                 495
Asn Gly Ile Pro Thr Arg Thr Asn Ile Gly Trp Met Val Ser Leu Arg
            500                 505                 510
Tyr Arg Asn Lys His Ile Cys Gly Gly Ser Leu Ile Lys Glu Ser Trp
        515                 520                 525
Val Leu Thr Ala Arg Gln Cys Phe Pro Ser Arg Asp Leu Lys Asp Tyr
    530                 535                 540
Glu Ala Trp Leu Gly Ile His Asp Val His Gly Arg Gly Asp Glu Lys
545                 550                 555                 560
Cys Lys Gln Val Leu Asn Val Ser Gln Leu Val Tyr Gly Pro Glu Gly
                565                 570                 575
Ser Asp Leu Val Leu Met Lys Leu Ala Arg Pro Ala Val Leu Asp Asp
            580                 585                 590

Phe Val Ser Thr Ile Asp Leu Pro Asn Tyr Gly Cys Thr Ile Pro Glu
        595                 600                 605
Lys Thr Ser Cys Ser Val Tyr Gly Trp Gly Tyr Thr Gly Leu Ile Asn
    610                 615                 620
Tyr Asp Gly Leu Leu Arg Val Ala His Leu Tyr Ile Met Gly Asn Glu
625                 630                 635                 640
Lys Cys Ser Gln His His Arg Gly Lys Val Thr Leu Asn Glu Ser Glu
                645                 650                 655
Ile Cys Ala Gly Ala Glu Lys Ile Gly Ser Gly Pro Cys Glu Gly Asp
            660                 665                 670
Tyr Gly Gly Pro Leu Val Cys Glu Gln His Lys Met Arg Met Val Leu
        675                 680                 685
Gly Val Ile Val Pro Gly Arg Gly Cys Ala Ile Pro Asn Arg Pro Gly
    690                 695                 700
Ile Phe Val Arg Val Ala Tyr Tyr Ala Lys Trp Ile His Lys Ile Ile
705                 710                 715                 720
Leu Thr Tyr Lys Val Pro Gln Ser Leu Lys Val Cys Leu Lys His Pro
                725                 730                 735
Pro Ile Gln Leu Ser Phe Thr Arg Phe Gln Arg Met Trp Asn Leu Lys
            740                 745                 750
Cys His Leu Gln Gln Ser Asp Asn Tyr Trp Arg Val Met Phe Val Glu
        755                 760                 765
Ile Leu Ile Asn Val Tyr Gly Cys Phe Leu Leu Phe Cys Leu Ser Val
    770                 775                 780
Leu Phe Cys Gln Cys Ser Glu Leu Arg Tyr Met Gln Val His Ile Ser
785                 790                 795                 800
Arg Tyr Leu Asn Gly Leu Lys Lys His Thr Gly Ile Phe Ala Gly
                805                 810                 815

## Description

### FIELD OF THE INVENTION

This invention relates to a novel protein of human origin and its production process. Particularly, it relates to a novel proteinous angiogenic factor of human origin, which enhances the growth of vascular endothelial cells but does not activate the growth of other cells such as smooth muscle cells, fibroblasts, hepatocytes and the like, and to a process for the production thereof.

### BACKGROUND OF THE INVENTION

Principal cells which constitute a blood vessel are vascular endothelial cells of tunica intima, smooth muscle cells of tunica media and fibroblasts of tunica externa. In addition, peripherally existing capillary blood vessels are composed solely of vascular endothelial cells. Though the mechanism of new formation of blood vessels, or angiogenesis, has not yet been elucidated in full details, it is considered that the angiogenesis starts firstly with dissolution of the blood vessel wall matrix and subsequent growth and migration of vascular endothelial cells.

Angiogenesis can be found during the prenatal period when new tissues and blood vessels are formed and at the time of the occurrence of physiological phenomena in the adult body such as periodical development of uterine endometrium and lutenization in ovaries, as well as under pathologic conditions such as chronic inflammation, wound healing and the like. New formation of blood vessels can also be found at the time of the growth of tumor cells. Endothelial cells which cover the inner wall of blood vessels are possessed of many physiological functions such as maintenance of anti-thrombotic activity, regulation of matter permeation, regulation of blood pressure and the like. In a patient suffering from a blood vessel-related disease such as arteriosclerosis, myocardial infarction or the like, abnormality can be found in these blood vessel-constituting cells.

A number of angiogenic factors have been found in the *in vivo* experimental systems for the formation of new blood vessels, such as an experiment in which chick chorio-allantoic membrane is used. For example, generally known proteinous angiogenic factors include basic fibroblast growth factor (bFGF), epidermal growth factor (EGF), platelet-derived growth factor (PDGF), transforming growth factor (TGF) and the like.

Though these prior art angiogenic factors having the ability to enhance formation of new blood vessels are possessed of the activity to enhance growth of vascular endothelial cells, these factors also strongly activate growth of other cells. For example, bFGF activates growth of various cells such as fibroblasts, smooth muscle cells, epidermal cells and the like. In consequence, each of these prior art angiogenic factors having a broad range of growth enhancing effects on various types of cells enhances not only the formation of new blood vessels but also the growth of other cells at the same time. In other words, these prior art factors have a problem of causing secondary reactions when used because of their inability to selectively enhance formation of new blood vessels.

Accordingly, the present invention contemplates overcoming the aforementioned problems involved in the prior art and, as the results, providing a purified angiogenic factor which enhances growth of vascular endothelial cells but does not or hardly activate growth of other cells such as smooth muscle cells, fibroblasts, hepatocytes and the like. The present invention also contemplates developing side effect-free pharmaceutical preparations and medical devices based on such a purified angiogenesis factor.

With the aim of accomplishing these objects, the inventors of the present invention have conducted intensive .studies and found that products of human ovarian tumor established cell lines, HUOCA-II and HUOCA-III, were able to enhance growth of vascular endothelial cells selectively. The results have been disclosed in Japanese Patent Application Kokai Nos. 2-261375, 2262523 and 3-84000.

Thereafter, the present inventors have carried out studies on the purification of the aforementioned products of HUOCA-II and HUOCA-III cell lines from their serum-free culture supernatants, making use of specific purification techniques, and have succeeded in obtaining a highly purified specific protein having the aforementioned desirable properties, that is, having a strong activity to enhance growth of vascular endothelial cells but with no activity to activate growth of other cells such as smooth muscle cells, fibroblasts, hepatocytes and the like.

By further continuing the studies,a total RNA was isolated from the HUOCA-II or HUOCA-III cells and its cDNA was cloned. Thereafter, the DNA sequence of the cDNA was determined and its corresponding amino acid sequence was deduced, thereby succeeding in obtaining the novel protein of the present invention.

### SUMMARY OF THE INVENTION

According to a first aspect of the present invention, there is provided a single chain protein produced by HUOCA-II or HUOCA-III, which has the following properties of:
(1) having a molecular weight, when determined by SDS polyacrylamide gel electrophoresis, of from 72,000 to 80,000 daltons under a non-reducing condition or from 79,000 to 85,000 daltons under a reducing condition;
(2) containing three peptide chains, respectively represented by the Sequence ID Nos. 1, 2 and 3 as attached hereto (in the Sequence ID No. 3, "Xaa" means an unidentified amino acid residue), in one molecule;
(3) having an activity to enhance the growth of vascular endothelial cells;
(4) having no activity to enhance the growth of fibroblasts; vascular smooth muscle cells and hepatocytes;
(5) having no activity to enhance or inhibit the browth of HeLa cells; and
(6) having an activity to enhance formation of new blood vessels.

According to a second aspect of the present invention, there is provided a protein of human origin which contains an amino acid sequence or a portion of the amino acid sequence represented by the Sequence ID No. 4 attached hereto that has been identified by isolating a corresponding total RNA molecule from HUOCA-II or HUOCA-III cells, cloning a cDNA corresponding to the proteins, determining the DNA sequence of the cDNA and deducing an amino acid sequence from the DNA sequence.

According to a third aspect of the present invention, there is provided a process for the production of a protein of human origin according to the first or second aspect of the present invention, which comprises purifying a serum-free culture supernatant of a human ovarian tumor cell or established cell line thereof, especially HUOCA-II or HUOCA-III, by an optional combination of purification techniques including (a) cation exchange chromatography, (b) heparin affinity chromatography, (c) heparin affinity high performance liquid chromatography and (d) reverse phase high performance liquid chromatography, or which comprises the steps of (i) preparing a DNA fragment containing a nucleotide sequence which encodes the protein or a portion of the protein shown in the Sequence ID No. 4 attached hereto, (ii) obtaining a transformant by transforming cells of a host with the DNA fragment prepared in the above step (i) or with a vector containing the DNA fragment and (iii) culturing the transformant obtained in the above step (ii) to allow the transformant to produce the protein of the Sequence ID No. 4, or a portion of the protein, subsequently recovering the protein from resulting culture mixture.

According to a fourth aspect of the present invention, there is provided a pharmaceutical preparation which contains the protein or a portion of the protein of the first and/or second aspect of the present invention as an active ingredient

According to a fifth aspect of the present invention, there is provided a DNA fragment or cDNA-fragment which contains a nucleotide sequence or a portion of the nucleotide sequence represented by the Sequence ID No. 5 attached hereto wherein at least one base may be substituted based on the degeneracy of genetic code.

According to a sixth aspect of the present invention, there is provided an expression vector containing the DNA fragment, as well as a transformant transformed with the DNA fragment or the expression vector.

Other objects and advantages of the present invention will be made apparent as the description progresses.

### BRIEF DESCRIPTION OF THE DRAWINGS

Fig. 1 is a graph showing the absorbance, measured at a wave length of 280 nm, of each eluate fraction resulting from the treatment of an HUOCA-III serum-free culture supernatant with cation exchange chromatography.

Fig. 2 is a graph showing the results of the measurement of activities in the eluate fractions obtained in Fig. 1 to enhance the growth of vascular endothelial cells.

Fig. 3 is a graph showing the absorbance, measured at a wave length of 280 nm, of each eluate fraction resulting from a heparin affinity chromatographic treatment of the active fractions of the cation exchange chromatography eluates having the vascular endothelial cell growth-enhancing activity.

Fig. 4 is a graph showing the results of the measurement of activities in the eluate fractions obtained in Fig. 3 to enhance the growth of vascular endothelial cells.

Fig. 5 is a graph showing the absorbance, measured at a wave length of 215 nm, of each eluate fraction resulting from a heparin affinity high performance liquid chromatographic treatment of the active fractions of the heparin affinity chromatography eluates having the vascular endothelial cell growth-enhancing activity.

Fig. 6 is a graph showing the results of the measurement of activities in the eluate fractions obtained in Fig. 5 to enhance growth of vascular endothelial cells.

Fig. 7 is a graph showing the absorbance, measured at a wave length of 215 nm, of each eluate fraction resulting from a reverse phase high performance liquid chromatographic treatment of the active fractions of the heparin affinity high performance liquid chromatography eluates having the vascular endothelial cell growth-enhancing activity.

Fig. 8 is a graph showing the results of the measurement of activities in the eluate fractions obtained in Fig. 7 to enhance the growth of vascular endothelial cells.

Fig. 9 is a graph showing an SDS polyacrylamide gel electrophoresis pattern of a highly purified product (glycoprotein) obtained in Example 1 of the present invention.

Fig. 10 is a graph showing results of the measurement of the vascular endothelial cell growth-enhancing activity of the highly purified product eluted from each cut portion of the electrophoresis gel of Fig. 9.

Fig. 11 is a graph showing an SDS-polyacrylamide gel electrophoresis pattern of an N-glycanase-treated product of the highly purified product (glycoprotein) obtained in Example 1 of the present invention.

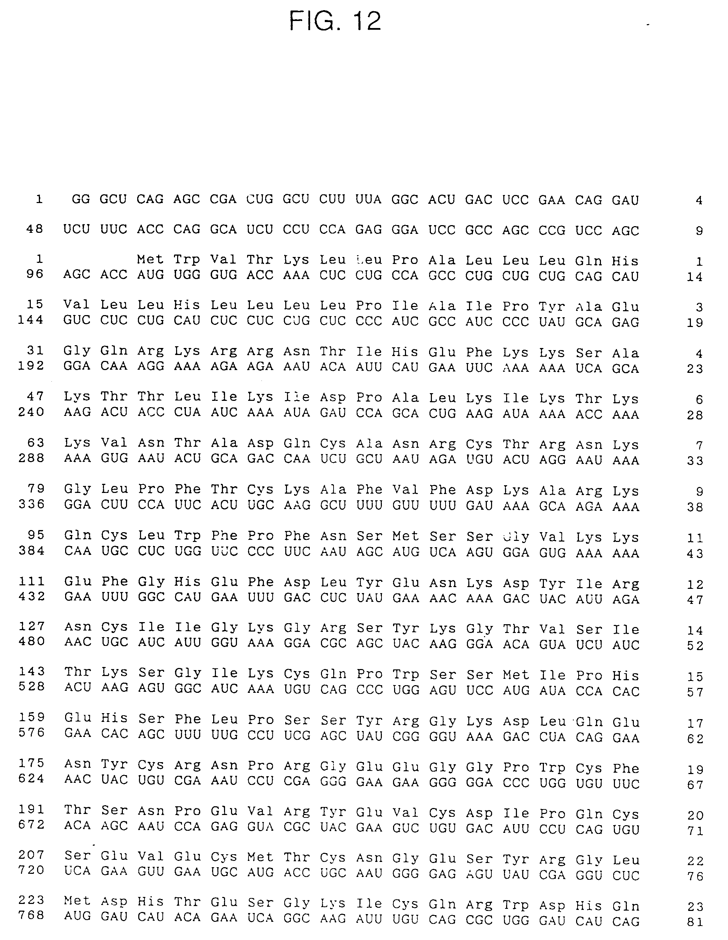

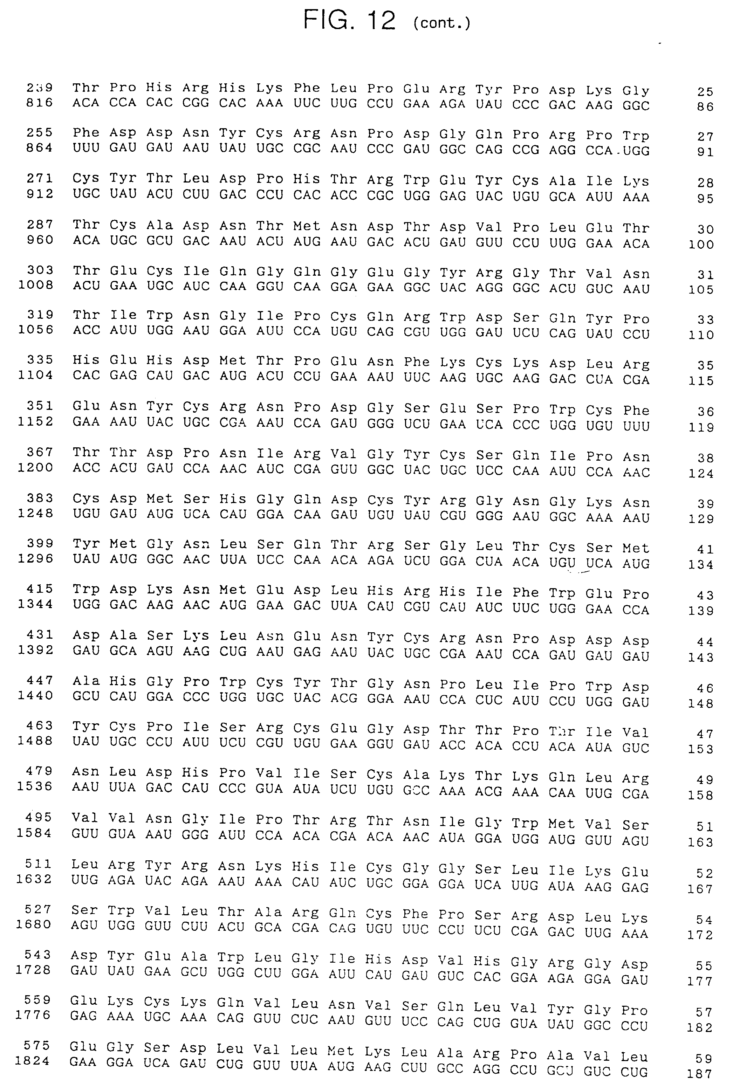

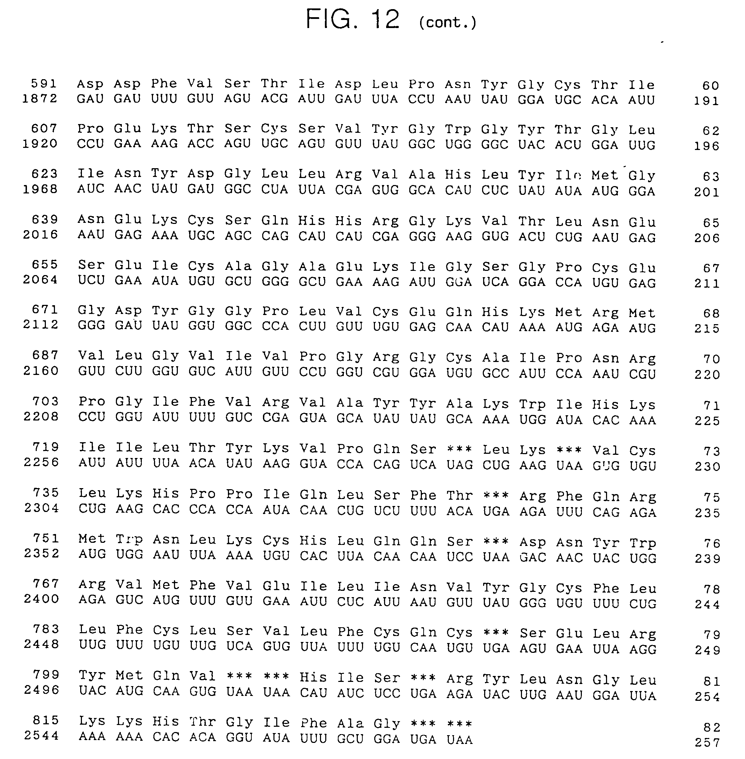

Fig. 12 represents the nucleotide sequence of the mRNA from which the cDNA obtained in Example 1 step (B) is translated and the corresponding amino acid sequence deduced from the nucleotide sequence.

### DETAILED DESCRIPTION OF THE INVENTION

Firstly, a first and a second aspects of the present invention are described in detail.

The gist of the first aspect of the present invention resides in a single chain protein produced by HUOCA-II or HUOCA-III, which has the following properties of:
(1) having a molecular weight, when determined by SDS polyacrylamide gel electrophoresis, of from 72,000 to 80,000 daltons under a non-reducing condition or from 79,000 to 85,000 daltons under a reducing condition;
(2) containing three peptide chains, respectively represented by the Sequence ID Nos. 1, 2 and 3 as attached hereto (in the Sequence ID No. 3, "Xaa" means an unidentified amino acid residue), in one molecule;
(3) having an activity to enhance the growth of vascular endothelial cells;
(4) having no activity to enhance the growth of fibroblasts, vascular smooth muscle cells and hepatocytes;
(5) having no activity to enhance or inhibit the growth of HeLa cells; and
(6) having an activity to enhance the formation of new blood vessels.

The gist of the second aspect of the present invention resides in a protein of human origin which contains an amino acid sequence or a portion of the sequence represented by the Sequence ID No. 4 attached hereto that has been identified by isolating a corresponding mRNA molecule from HUOCA-II or HUOCA-III cells, cloning a gene corresponding to the mRNA, determining the DNA sequence of the gene and deducing an amino acid sequence from the DNA sequence.

The human ovarian tumor established cell lines HUOCA-II and HUOCA-III have been deposited by the present inventors on March 1, 1989, in Fermentation Research Institute, Agency of Industrial Science and Technology, and have been assigned the designations as FERM BP-2310 and FERM BP-2311. Though culturing of the HUOCA-II and HUOCA-III and preparation of their serum-free culture supernatants may be carried out in the usual way, these techniques are disclosed in detail by the present inventors in Japanese Patent Application Kokai Nos. 2-261375, 2-262523 and 3-84000.

The protein of the present invention comprises a single chain protein molecule, and the single chain protein contains three peptide chains respectively represented by the Sequence ID Nos. 1, 2 and 3 as attached hereto.

The protein of the present invention may be prepared from a serum-free culture supernatant of the human ovarian tumor established cell line, HUOCA-II or HUOCA-III, by subjecting the supernatant to a series of purification steps including (a) cation exchange chromatography, (b) heparin affinity chromatography, (c) heparin affinity high-performance liquid chromatography and (d) reverse-phase high-performance liquid chromatography. Preferably, it may be prepared in accordance with the following illustrative steps (i) to (iv).

### Preparation of protein

(i) A serum-free culture supernatant of HUOCA-II or HUOCA-III is adsorbed on to a cation exchange resin packed in a column. In this instance, the cation exchange resin may be either strongly ionic or weakly ionic, but the use of S-Sepharose® (trademark of Pharmacia) is particularly preferred. The thus adsorbed portion onto a cation exchange resin in the column is washed with an appropriate buffer solution and then subjected to a linear gradient elution using two buffer solutions respectively containing 150 mM NaCl and 2 M NaCl to collect active fractions showing the activity to enhance the growth of vascular endothelial cells [step (a)].
(ii) The active fractions obtained in the above step (i) are pooled and diluted by a factor of 2 to 3 with the same buffer solution containing 150 mM of NaCl. The thus diluted sample is applied to a heparin-Sepharose column, washed with the same buffer solution containing 0.5 M NaCl and then subjected to a linear gradient elution using two buffer solutions respectively containing 0.5 M NaCl and 2 M NaCl to collect active fractions showing the activity to enhance the growth of vascular endothelial cells [step (b)].
(iii) The active fractions obtained in the above step (ii) are diluted in the same manner, applied to a heparin column for high performance liquid chromatography use and then subjected to elution in the same manner to collect active fractions showing the activity to enhance the growth of vascular endothelial cells [step (c)].
(iv) The active fractions obtained in the above step (iii) are applied to a column for reverse-phase high-performance liquid chromatography use to obtain a purified product (protein) having the activity to enhance the growth of vascular endothelial cells [step (d)].

Any usually used buffer solution such as a phosphate buffer or the like may be used in the above glycoprotein preparation steps, and Sepharose or any other general purpose carrier may be used as a carrier of heparin.

The thus purified product has been identified as a glycoprotein, namely a sugar chain-attached protein molecule, on the basis of the facts that (1), when the purified product was allowed to react with a sugar chain-hydrolyzing enzyme N-glycanase and the resulting product was analyzed by 0.1% SDS-containing 10% polyacrylamide gel electrophoresis, the electrophoresis pattern of the thus treated product showed a decreased molecular weight level due to the digestion of sugar chains and (2) the purified product showed an affinity for concanavalin A.

In addition, the protein portion of the glycoprotein of the present invention was identified as a single chain protein molecule, because the purified product showed a single band when analyzed by 0.1% SDS-containing 10% polyacrylamide gel electrophoresis under reducing conditions.

Though the amino acid sequence of the protein portion of the thus obtained glycoprotein could be determined by any usually used means, the following illustrative steps (1) to (3) were employed herein in that order.

### Determination of amino acid sequence

### (1) Reductive carboxymethylation

The sample purified and isolated in the aforementioned step (iv) by reverse-phase high-performance liquid chromatography was concentrated using a concentrator and eluted with an eluting solution consisting of 8 M urea, 0.5 M Tris-HCl pH 8.0 and 1 mM EDTA. To this was added dithiothreitol to a final concentration of 20 mM. After nitrogen gas flush, the reduction reaction was carried out in the dark for 2 hours at room temperature. Thereafter, monoiodoacetic acid was added to the resulting reaction mixture to a final concentration of 20 mM, and the alkylation reaction was carried out in the dark for 30 minutes at room temperature.

### (2) Digestion with lysyl endopeptidase

The reductive alkylation product obtained in the above step (1) was mixed with 2-mercaptoethanol, followed by the addition of 0.1 N NaOH to adjust the mixture to pH 8.5. Lysyl endopeptidase (Wako Pure Chemical Industries, Ltd.) was added in a 1:10 (w/w) ratio to the thus prepared substrate to carry out the enzymatic hydrolysis reaction at 37°C for 4 hours.

### (3) Fractionation of peptide fragments and determination of the amino acid sequence

The peptide fragments mixture obtained in the above step (2) were separated by reverse-phase high-performance chromatography using an RP300 column (Applied Biosystems, Inc.). The elution was carried out by linear concentration gradient of acetonitrile from 0% to 60% in the presence of 0.1% TFA. The thus obtained peptide fragments by the elution treatment were subjected to Edman degradation using a gas phase sequencer (Model 477A; Applied Biosystems, Inc.), and the resulting PTH-amino acids were identified using a high-performance liquid chromatography for PTH-amino acid identification use (Model 120A; Applied Biosystems, Inc.). As the results, it was found that the protein portion of the glycoprotein of the present invention contained three peptide chains respectively represented by the Sequence ID Nos. 1, 2 and 3.

### Determination of the complete DNA sequence by PCR

The amino acid sequence determined in the above step (3) coincided well with that of human hepatocyte growth factor (hHGF). With regard to hHGF, its cDNA sequence has been reported by Nakamura (*Nature*, vol.342, pp.440 - 443, 1989) and Miyazawa (*Biochemical and Biophysical Research Communication*, vol.163, pp.967 - 973, 1989).

Since several cDNA nucleotide sequences have been reported on the hHGF family, primers for PCR use were prepared using a DNA synthesizer based on the common sequences in the 5' and 3' non-translation regions of these known nucleotide sequences. That is, primers were synthesized based on a region including 47 to 82 position bases (5' primer) counting in upstream direction from the 5' end of the translation region (translation initiation point) and another region including 1 to 37 position bases (3' primer) counting in downstream direction from the 3' end.

The total RNA sample was prepared from the human ovarian tumor cell line HUOCA-III by means of an SDS-phenol method. Using the thus prepared total RNA as a template, cDNA synthesis was carried out making use of M-MLV reverse transcriptase. The thus synthesized cDNA was subjected to PCR and the resulting PCR product was applied to agarose gel electrophoresis to find a DNA fragment having a size of about 2.3 kb. Since the open reading frame of the HGF family so far reported has a size of about 2.3 kb, this DNA fragment was considered to be a cDNA molecule coding for the HUOCA-III-originated novel protein of the present invention. In consequence, this DNA fragment was purified from the agarose gel, inserted into the pUC18 plasmid vector and then transformed into *Escherichia coli* JM109. Some of the thus obtained clones were examined making use of the dideoxy method to determine their nucleotide sequences. By correcting reading errors at the time of the PCR study, a nucleotide sequence corresponding to the novel protein of HUOCA-III origin was determined. The thus determined nucleotide sequence is shown in the Sequence ID No. 5 attached hereto, and an amino acid sequence deduced from the nucleotide sequence in the Sequence ID No. 4

### Measurement of molecular weight by SDS-polyacrylamide gel electrophoresis

Electrophoresis was carried out using a 10% polyacrylamide gel in accordance with the procedure of Lammeli *et al*. (*Nature*, vol.277, pp.680 - 685, 1970). The resulting gel was fixed by treating it with 50% ethanol and 40% acetic acid for 30 minutes, washed with 10% ethanol and 5% acetic acid and then subjected to silver staining. The protein of the present invention was stained as a single band, and its molecular weight was estimated to be about 72,000 to 80,000 daltons based on its relative mobility. In addition, another electrophoresis was carried out under a reducing condition by adding 2-mercaptoethanol to the sample to a concentration of 5% and treating the mixture at 95°C for 10 minutes, followed by the same procedure as the case of the above non-reducing condition. Under the reducing condition, the molecular weight of the protein of the present invention was estimated to be about 79,000 to 85,000 daltons.

Next, a third aspect of the present invention is described in the following.

The gist of the third aspect of the present invention resides in a process for the production of the protein of the first or second aspect of the present invention.

Firstly, a culture mixture containing the protein of the first or second aspect of the present invention is obtained.

The single chain protein of the first aspect of the present invention is obtained by recovering it from a serum-free culture supernatant of the human ovarian tumor cell line, HUOCA-II or HUOCA-III

The novel protein of the second aspect of the present invention is obtained by preparing a DNA fragment containing a nucleotide sequence which encodes the novel protein represented by the amino acid sequence or a portion of the sequence shown in the Sequence ID No. 4, preferably the DNA fragment or a portion of the DNA fragment represented by the Sequence ID No. 5, transforming appropriate host cells with the thus ligated fragment directly or indirectly using a proper expression vector, culturing the thus obtained transformant and then recovering the novel protein of the Sequence ID No. 4 from the resulting culture mixture.

The recovering step may be effected, though not particularly limited, by purifying the novel protein by means of (a) cation exchange chromatography, (b) heparin affinity chromatography, (c) heparin affinity high-performance liquid chromatography and (d) reverse-phase high-performance liquid chromatography, in any optional combination or order.

According to a fourth aspect of the present invention, there is provided a pharmaceutical preparation which contains the protein of the first and/or second aspect of the present invention as an active ingredient.

The pharmaceutical preparation may be applied to various dosage forms such as tablets, sugar coated tablets, powders, capsules, granules, suspensions, emulsions, parenteral solutions, external preparations, ointments and the like, using the preparation alone or together with other necessary ingredients in combination with appropriate carriers, fillers and the like.

The protein of the present invention is possessed of a function to enhance vascular endothelial cell growth in human and various animals, but does not enhance the growth of fibroblasts, vascular smooth muscle cells or hepatocytes in human and animals and does not enhance of inhibit the growth of HeLa cells. Because of such nature, the growth of vascular endothelial cells can be enhanced selectively and, as the results, new formation of blood vessels can be effected smoothly without causing secondary reactions.

The term "it does not enhance the growth of fibroblasts; vascular smooth muscle cells or hepatocytes and does not enhance or inhibit the growth of HeLa cells" as used herein includes two cases; one case meaning that it does not enhance the growth of fibroblasts, vascular smooth muscle cells or hepatocytes and does not enhance or inhibit the growth of HeLa cells at all, and the other case meaning that it shows these activities to some extent but to an extremely small degree in comparison with its activity to enhance the growth of vascular endothelial cells.

Illustrative procedures for the measurement of activities of the protein of the present invention to enhance the growth of vascular endothelial cells, fibroblasts, vascular smooth muscle cells, hepatocytes and HeLa cells and to inhibit the growth of HeLa cells will be described later in detail in Examples.

In addition to the above properties, the protein of the present invention shows an affinity for concanavalin A. In the present invention, the affinity for concanavalin A was examined in the following manner.

### Measurement of affinity for concanavalin A

Using a dot blot apparatus (BioDot; Bio-Rad Laboratories, Inc.), a 500 ng portion of the purified product described in the foregoing was adsorbed to a nitrocellulose membrane (Bio-Rad Laboratories, Inc.) which has in advance been soaked in 10 mM Tris-HCl buffer (pH 7.5) containing 0.15 M NaCl. After air-drying, the resulting membrane was washed by soaking it for 10 minutes in 10 mM Tris-HCl buffer (pH 7.5) containing 0.15 M NaCl and 0.05% Tween and then replacing the washing buffer by a fresh one. After repeating the washing step 4 times, the membrane was soaked for 1 hour at 4°C in the same buffer which has been further supplemented with 1% BSA (bovine serum albumin), and washed again.

The thus treated membrane was soaked in a solution containing 10 µg/ml of labelled horseradish peroxidase (HRP) - concanavalin A at 4°C for 1 hour and washed again. Thereafter, the HRP remaining after the washing was allowed to perform a coloring reaction in the presence of H₂O₂ using 3,3'-diaminobenzidine as a substrate, in order to judge the affinity of the inventive protein for concanavalin A. As the results, the purified product blotted on the membrane showed development of a brown color, while a control test resulted in no coloration, thus confirming the affinity of the purified product for concanavalin A.

As described in the foregoing, the protein of the present invention is possessed of excellent ability to enhance vascular endothelial cells growth as well as its function to enhance new formation of blood vessels. Because of such nature, a physiologically active pharmaceutical preparation containing the inventive protein can be used as a healing enhancer of wound, burn injury, decubitus, postoperative tissue damage or the like or as a drug for the treatment of cardiac angiopathy, as well as its application to artificial organs such as artificial blood vessel, artificial skin and the like. In addition, antibodies specific for the protein of the present invention and inhibitors of the inventive protein can be used effectively as diagnostic and therapeutic drugs of malignant tumor, retinopathy, chronic rheumatoid arthritis and the like.

### EXAMPLES

The following examples are provided to further illustrate the preparation process of the protein of the present invention, the measurement of its molecular weight, its activities on various cells and the presence or absence of its sugar chain moiety. It is to be understood, however, that the examples are for purpose of illustration only and are not intended as a definition of the limits of the invention.

### Example 1

### (A) Preparation of the protein, measurement of its molecular weight and determination of its aminoacid sequence

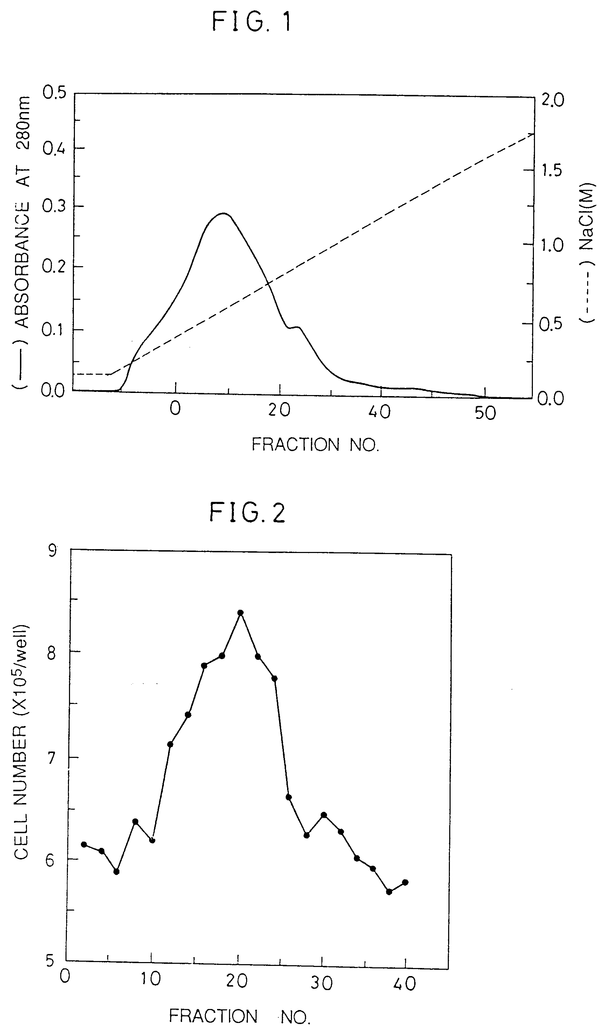

(1) To 10 liters of HUOCA-III serum-free culture supernatant was added CHAPS (3-[(3-cholamidopropyl)dimethylammonio]-1-propanesulfonate; Dojin Kagaku K.K.) to a final concentration of 0.03%. The thus prepared serum-free culture supernatant was applied to a 40 ml volume of S-Sepharose (Fast Flow, Pharmacia) which has been equilibrated in advance with 10 mM phosphate buffer (pH 7.2) containing 0.15 M NaCl and 0.03% CHAPS, and the contents were adsorbed at a flow rate of 200 ml/hour at 4°C. After washing with the just described buffer solution containing 0.15 M NaCl, the adsorbed contents were eluted by a linear NaCl gradient using two buffers containing 0.15 M NaCl and 2.0 M NaCl at a flow rate of 200 ml/hour and at a temperature of 4°C. The eluate was checked for its absorbance at 280 nm and collected as fractions of 6.7 ml/tube. Results of the absorbance measurement at 280 nm are shown in Fig. 1.

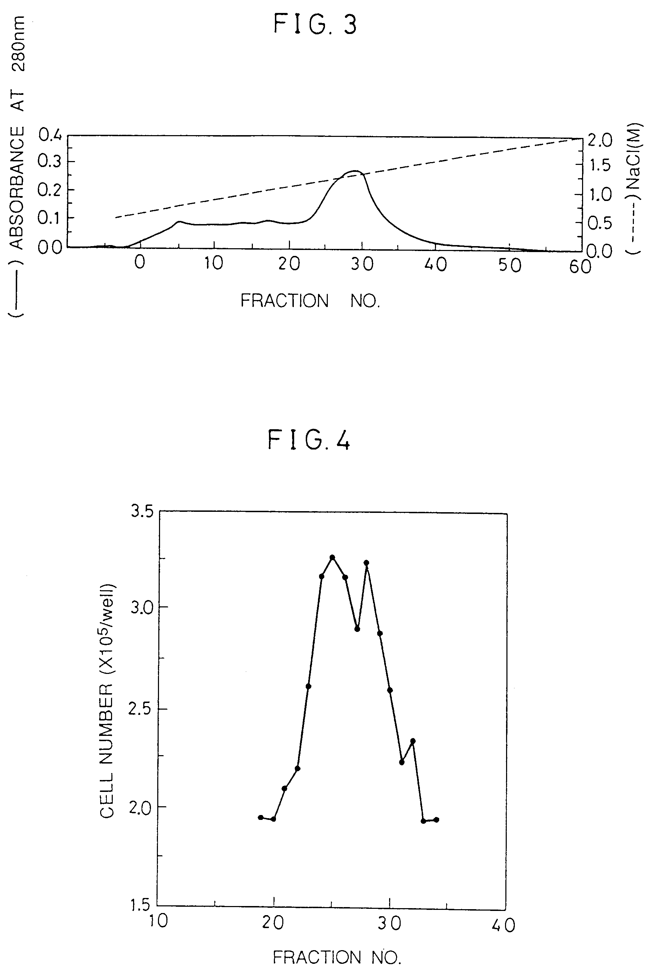

Each of the thus collected fractions was checked for its activity to enhance the growth of bovine aorta endothelial cells in the following manner. As shown in Fig. 2, the cell growth enhancing activity was found mostly in fractions 12 to 24.

### Measurement of activity to enhance the growth of bovine aorta endothélial cells

Bovine aorta endothelial cells were suspended in DME (Dulbecco's Modified Eagle's) medium (Flow Laboratories, Inc.) which has been supplemented with 10% fetal calf serum, and the cell suspension was poured in a 24 well multi-dish (Corning Glassworks) with a density of 5 x 10³ cells/well. On the following day, the medium was replaced by fresh DME medium containing 5% fetal calf serum, and a sample to be tested was added to the fresh medium, followed by 4 days of culturing to measure the number of resulting cells.
(2) The fractions obtained in the above step (1) having high vascular endothelial cell growth-enhancing activities were pooled and diluted with a buffer solution by a factor of 3, and the contents were adsorbed to heparin-Sepharose CL-6B (Pharmacia; bed volume, 4 ml) which has been equilibrated in advance with a buffer solution containing 0.5 M NaCl, at a flow rate of from 0.2 to 0.4 ml/minute and at a temperature of 4°C. After washing with the same buffer solution containing 0.5 M NaCl, the adsorbed contents were eluted by a linear NaCl gradient using two buffers containing 0.5 M NaCl and 2.0 M NaCl at a flow rate of 0.2 ml/min and at a temperature of 4°C. The eluate was checked for its absorbance at 280 nm and collected as fractions of 3 ml/tube. Results of the absorbance measurement at 280 nm are shown in Fig. 3.

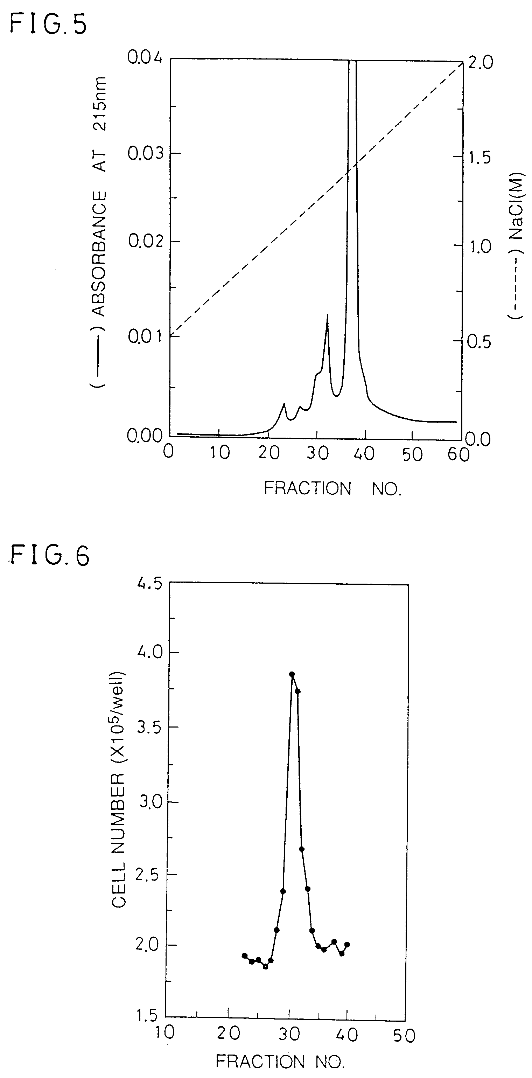

Each of the thus collected fractions was checked for its activity to enhance the growth of bovine aorta endothelial cells in the same manner as described above. As shown in fig. 4, the cell growth enhancing activity was found mostly in fractions 23 to 30.
(3) The fractions obtained in the above step (2) having high vascular endothelial cell growth-enhancing activities were pooled and diluted with a buffer solution by a factor of 3, and the contents were adsorbed on to a TSK-heparin 5PW column (7.5 mm in inside diameter and 7.5 cm in length; Tosoh Corp.) which has been equilibrated in advance with a buffer solution containing 0.5 M NaCl. After washing with the same buffer solution containing 0.5 M NaCl, the adsorbed contents were eluted by a linear NaCl gradient using two buffers containing 0.5 M NaCl and 2.0 M NaCl, at a flow rate of 0.5 ml/min and at room temperature. The eluate was checked for its absorbance at 215 nm and collected as fractions of 0.5 ml/tube. Results of the absorbance measurement at 215 nm are shown in Fig. 5.

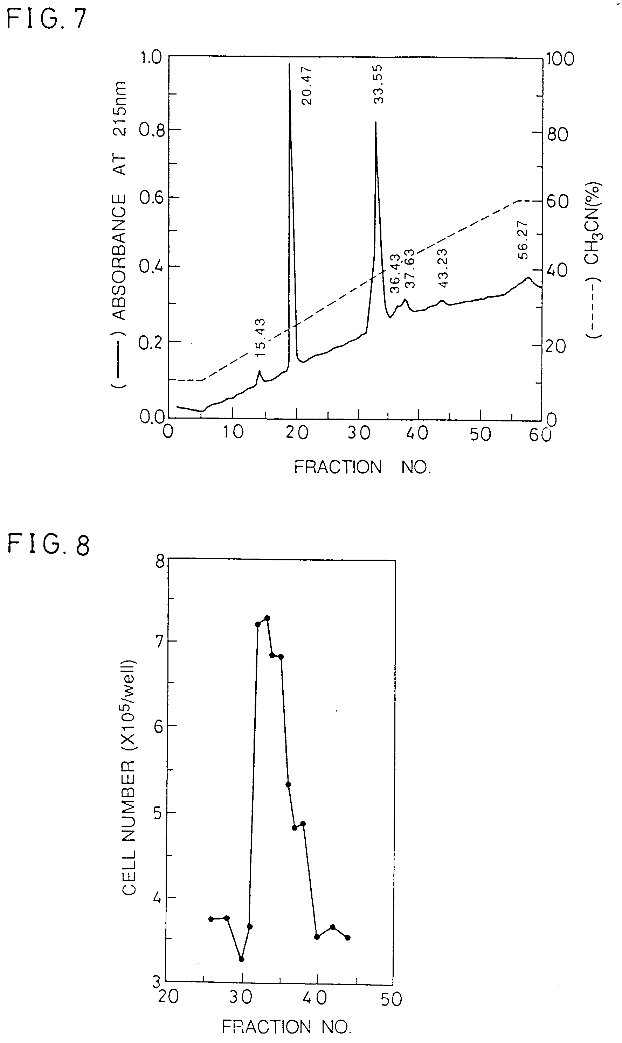

Each of the thus collected fractions was checked for its activity to enhance the growth of bovine aorta endothelial cells in the same manner as described above. As shown in Fig. 6, the cell growth enhancing activity was found mostly in fractions 30 to 32.
(4) The fractions obtained in the above step (3) having high vascular endothelial cell growth-enhancing activities were pooled and subjected to reverse phase chromatography using a vp-318 column (4.6 mm in inside diameter and 30 mm in length; Senshu Kagaku Co., Ltd.). In the presence of 0.1% trifluoroacetic acid (TFA), a linear gradient elution was carried out by increasing the concentration of acetonitrile from 10% to 60%, at a flow rate of 1.0 ml/min. The eluate was checked for its absorbance at 215 nm and collected as fractions of 10 ml/tube. Results of the absorbance measurement at 215 nm are shown in Fig. 7.

Each of the thus collected fractions was checked for its activity to enhance the growth of bovine aorta endothelial cells in the same manner as described above, with the results shown in Fig. 8. By collecting peak fractions, a highly purified product having high vascular endothelial cell growth-enhancing activity was obtained.
(5)The molecular weight of the highly purified product obtained in the above step (4) was measured by SDS polyacrylamide gel electrophoresis.
   The following 6 authentic samples whose molecular weights have been confirmed were used as molecular weight markers, and the electrophoresis was carried out in the same manner as described in the foregoing.

| [Molecular weight markers] | |
|---|---|
| 1. Rabbit muscle phosphorylase | (M.W., 97,400 daltons) |
| 2. Bovine serum albumin | (M.W., 66,200 daltons) |
| 3. Ovalbumin | (M.W., 45,000 daltons) |
| 4. Carbonic anhydrase | (M.W., 31,000 daltons) |
| 5. Soybean trypsin inhibitor | (M.W., 21,500 daltons) |
| 6. Lysozyme | (M.W., 14,400 daltons) |

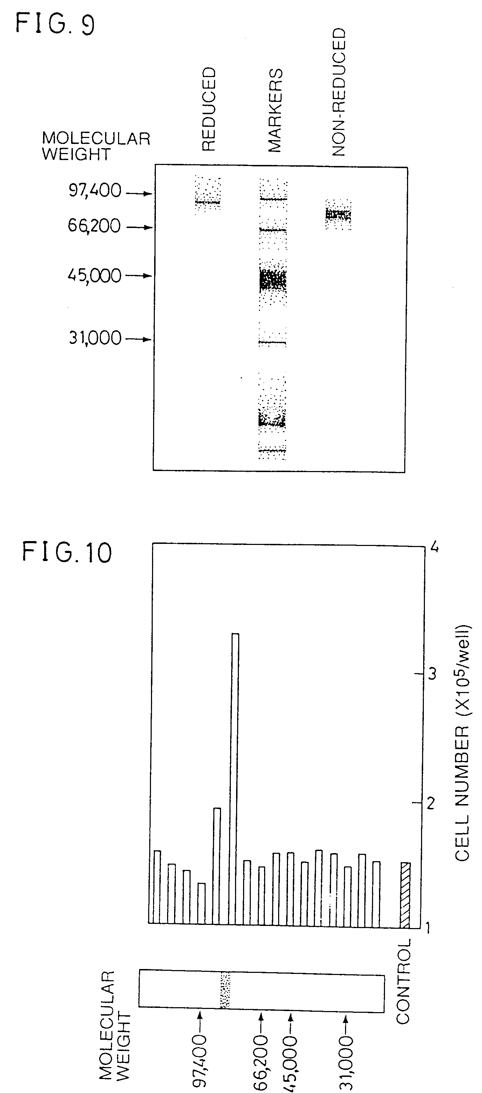

The thus obtained electrophoresis pattern is shown in Fig. 9. As is evident from the figure, the highly purified product obtained in the above step (4) has a molecular weight of 72,000 to 80,000 daltons under non-reducing condition, or 79,000 to 85,000 daltons under reducing condition, when measured by SDS polyacrylamide gel electrophoresis. It is evident also that the purified product is a single chain protein.

After the electrophoresis, the gel was cut out at intervals of 2 mm. Each of the thus cut portions was put into a test tube, ground into pieces, mixed with 500 µl of a buffer solution 0.03% CHAPS, 20 mmol PB pH 7.2 and then shaken at 4°C for 16 hours. The resulting mixture was centrifuged to recover supernatant fluid which was subsequently dialyzed against a buffer solution 0.03% CHAPS, 20 mmol PB pH 7.2. Contents in the thus dialyzed solution was freeze-dried and then dissolved in 100 µl of a buffer solution 0.03% CHAPS, 20 mmol PB pH 7.2 to measure the activity to enhance the growth of bovine aorta endothelial cells in the same manner as described in the foregoing. As shown in Figure 10, the endothelial cell growth-enhancing activity was observed in 72,000-80,000 molecular weight fraction obtained under non-reducing condition.

When the amino acid sequence of the highly purified product was determined in accordance with the procedure described in the foregoing, it was confirmed that the product contained three peptide chains respectively represented by the Sequence ID Nos. 1, 2 and 3.

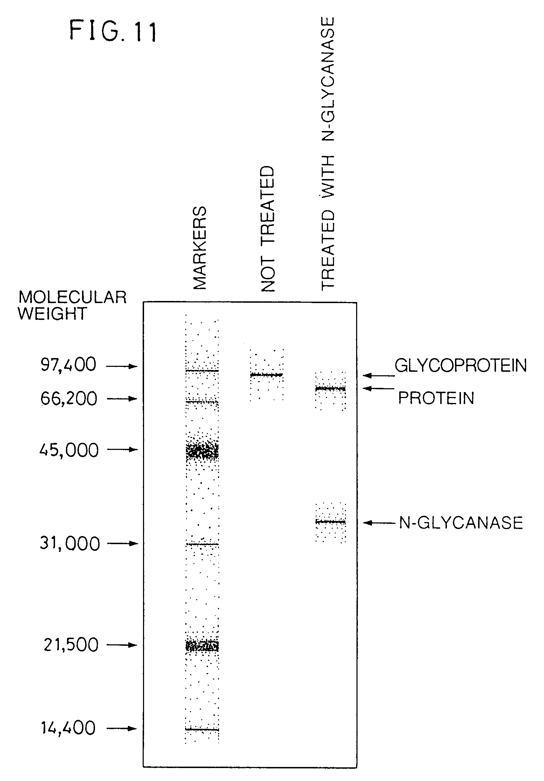

Also, in order to confirm the addition of sugar chains to the highly purified product, 5 µl (250 ng) of the high purity product and 3.2 µl of N-glycanase (Genzyme Corp.; 250 units/ml) were added to 30 µl of 50 mM Tris-HCl buffer (pH 8.0). After 18 hours of reaction, the resulting mixture was subjected to 0.1% SDS-10% polyacrylamide gel electrophoresis, followed by silver staining. As shown in Fig. 11, the resulting electrophoresis pattern clearly indicated a decrease in the molecular weight of the N-glycanase-treated product due to the separation of sugar chains.

### (B) Cloning of the DNA and estimation of the amino acid sequence

### (a) Synthesis of the cCNA

A 5 µl portion of the total RNA sample (10 µg/µl) which has been prepared from the human ovarian tumor cell line HUOCA-III by the SDS-phenol method was incubated at 70°C for 5 minutes and then cooled down rapidly. After 5 minutes of cooling on an ice bath, to this were added 10 µl of a 5 x buffer solution for reverse transcription use (250 mM Tris-HCl/pH 8.3, 375 mM KCl, 15 mM MgCl2), 15 µl of 2.5 mM dNTP (a mixture of dATP, dCTP, dGTP and dTTP; Takara Shuzo Co., Ltd.), 0.5 µl of 1 M DTT (dithiothreitol), 1 µl of oligo(dT)₁₂₋₁₈ (Amersham), 2.5 µl of a ribonuclease inhibitor (200 U/µl, Takara Shuzo Co., Ltd.), 13 µl of distilled water and 3 µl of M-MLV reverse transcriptase (200 U/µl, GIBCO-BRL). The thus prepared mixture was incubated at 37°C for 1 hour to effect cDNA synthesis. After removing the proteinous materials from the resulting reaction mixture by phenol treatment, the cDNA of interest was recovered by ethanol precipitation, dissolved in 50 µl of distilled water and then stored at -80°C.

### (b) Amplification of the cDNA which encodes the HUOCA-III-originated novel protein by polymerase chain reaction (PCR)

To 5 µl of the cDNA aqueous solution were added 70 µl of distilled water, 10 µl of a 10 x buffer solution for PCR use (500 mM KCl, 15 mM MgCl2, 100 mM Tris-HCl/pH 8.3, 0.01% (w/v) gelatin), 8 µl of dNTP (Takara Shuzo Co., Ltd.), 3 µl of a 5' primer (5' TCTTTTAGGCACTGACTCCGAACAGGATTCTTTCAC 3', 1 µg/µl) and 3 µl of a 3' primer (5' GTTGTATTGGTGGATCCTTCAGACACACTTACTTCAG 3'). The thus prepared mixture was incubated at 95°C for 7 minutes, followed by rapid cooling. The thus treated solution was mixed with 1 µl of Ampli Taq DNA polymerase (5 U/µl, Perkin Elmer Cetus), and the surface of the reaction solution was covered with mineral oil (nujol mineral oil manufactured by Perkin Elmer Cetus). Thereafter, PCR was carried out by 30 repetitions of a three step reaction (94°C for 1 minute, 60°C for 2 minutes and 72°C for 3 minutes). After completion of the reaction, mineral oil was removed by chloroform treatment, proteinous materials were removed by phenol treatment and then the PCR product was recovered by ethanol precipitation.

### (c) Digestion of the PCR product with BamHI

An 85 µl portion of the PCR product was mixed with 10 µl of a 10 x buffer solution for *Bam*HI reaction use (1.5 M NaCl, 60 mM Tris-HCl/pH 7.9, 60 mM MgCl2) and 5 µl of an aqueous solution of *Bam*HI (15 U/µl, Nippon Gene), and the resulting mixture was incubated at 37°C for 1 hour.

### (d) Purification of the BamHI-digested PCR product

The PCR product thus digested with *Bam*HI was subjected to 0.7% agarose gel electrophoresis at a constant voltage (100 V). After completion of the electrophoresis, the gel was stained with ethidium bromide to observe DNA bands using a UV transilluminator. A portion of the gel where a DNA band of 2.3 kb was observed was cut out, and the PCR product in the cut portion was purified using Sephaglas Band Prep Kit (Pharmacia).

### (e) Digestion of the pUC18 plasmid vector with BamHI

A 2 µl portion of pUC18 solution (1 µg/µl, Takara Shuzo Co., Ltd.) was mixed with 6.6 µl of distilled water, 3 µl of the 10 x buffer solution for *Bam*HI reaction use and 1.4 µl of *Bam*HI (15 U/µl, Nippon Gene), and the resulting mixture was incubated at 37°C for 1 hour to digest the plasmid. After completion of the reaction, proteinous materials were removed by phenol treatment and the thus digested plasmid fragments were recovered by ethanol precipitation. The thus recovered plasmid fragments were dissolved in 33 µl of distilled water and mixed with 4 µl of CIP buffer (50 mM Tris-HCl/pH 8.0, 1 mM MgCl₂) and 3 µl of alkaline phosphatase (calf intestine origin, 2,500 U/ml, Toyobo Co., Ltd.). The resulting mixture was incubated at 37°C for 40 minutes and then at 50°C for 20 minutes. After completion of the reaction, the *Bam*HI-digested fragments of the plasmid vector pUC18 were recovered by phenol treatment and subsequent ethanol treatment.

### (f) Transformation of E.Coli JM109 with the PCR product

To 6 µl (30 µg) of the the *Bam*HI-digested PCR product were added 2 µl (200 µg) of the pUC18 digest prepared in the above step (e), 2 µl of a 10 x ligation buffer solution (10 mM ATP, 200 mM DTT, 100 mM MgCl₂, 500 mM Tris-HCl/pH 7.9), 9 µl of distilled water and 1 µl of T4 DNA ligase (500 U/µl, Nippon Gene). After overnight reaction at 16°C, a portion of the resulting reaction solution was added to 100 µl of a suspension of *E. coli* JM109 competent cells (Nippon Gene). The thus prepared mixture was allowed to stand still for 20 minutes on an ice bath, heat-treated at 42°C for 45 seconds and then allowed again to stand still on an ice bath for at least 2 minutes. The thus treated mixture was added to 400 µl of High-compitence broth (Nippon Gene) and stirred on a shaker at 37°C for 60 minutes. To this were added 40 µl of 2% X-Gal (5-bromo-4-chloro-3-indolyl-β-D-galactopyranoside) dissolved in diethylformamide and 40 µl of 100 mM IPTG (isopropyl-β-D-thio-galactopyranoside). The thus prepared mixture was poured on LB plate medium (0.5% yeast extract, 1% Bacto-Trypton, 1.5% agar, 1% NaCl, 50 µg/ml ampicillin, pH 7.5) and incubated overnight at 37°C to find white (recombinant) colonies and blue (non-recombinant) colonies grown on the medium. By isolating white colonies, a JM109 transformant into which the cDNA of interest has been inserted was selected.

### (g) Preparation of the plasmid

The plasmid-introduced JM109 was cultured overnight at 37°C in 100 ml of LB medium (1% Bacto-Trypton, 0.5% yeast extract, 1% NaCl, pH 7.5). When the cells reached their logarithmic growth phase, they were collected by centrifugation (5 minutes, 5,000 rpm, 0°C) and suspended in 4 ml of P1 buffer solution (100 µg/ml RNase A, 50 mM Tris-HCl/pH 8.0, 10 mM EDTA). The resulting cell suspension was mixed with 4 ml of P2 buffer solution (200 mM NaOH, 1% SDS) to carry out an alkali treatment at room temperature for 5 minutes. After the alkali denaturation, the resulting mixture was neutralized by adding 4 ml of P3 buffer solution (2.55 mM Potassium acetate, pH 4.8) and then centrifuged at 15,000 rpm for 30 minutes at 4°C. The thus obtained supernatant fluid was applied to a QIAGEN-MIDI column-pack 100 (DIAGEN) which has been equilibrated in advance with 2 ml of QB buffer solution (750 mM NaCl, 50 mM MOPS [3-(N-morpholino)propanesulfonic acid]/pH 7.0, 15% ethanol). After washing the column twice with 4 ml of QC buffer solution (1 M NaCl, 50 mM MOPS/pH 7.0, 15% ethanol), the plasmid was eluted with 2 ml of QF buffer solution (1.2 M NaCl, 15% ethanol, 50 mM MOPS/pH 8.0). The eluate was mixed with 500 µl of isopropanol and centrifuged at room temperature for 30 minutes. Thereafter, the precipitate thus obtained was washed with 70% ethanol and dissolved in 100 µl of distilled water.

### (h) Determination of the nucleotide sequence by the dideoxy method

A 16 µl (3 µg) portion of the plasmid solution prepared in the above step (g) was mixed with 2 µl of 2 N NaOH and 2 µl of 2 mM EDTA, and the mixture was incubated at 37°C for 25 minutes to denature the plasmid. After the alkali denaturation, the resulting solution was mixed with 2 µl of 3 M sodium acetate and 100 µl of cold ethanol, and ethanol precipitation was effected by maintaining the mixture for 10 minutes at -80°C. The thus precipitated plasmid was recovered by centrifugation, washed with 70% ethanol and then dissolved in 7 µl of distilled water. To this were added 1 µl of a primer (0.5 pmole) and 2 µl of a 5 x buffer solution A (250 mM NaCl, 200 mM Tris-HCl/pH 7.5, 100 mM MgCl2). After 2 minutes of incubation at 65°C, the resulting solution was gradually cooled down to 30°C to effect annealing of the denatured plasmid and the primer. To the resulting solution were added 1 µl of 0.1 M dithiothreitol, 2 µl of a labeling mixture (1.5 µM 7-deaza-dGTP, 1.5 µM dATP, 1.5 µM dTTP), 0.5 µl of [α-³⁵S]dCTP (1,000 Ci/mmole, Amersham) and 2 µl of Sequenase Ver. 2.0 (1.5 U/µl, United States Biochemical Corporation). After 5 minutes of reaction at 37°C, a 3.5 µl portion of the resulting reaction mixture was added to 2.5 µl of each of a G solution (80 µM 7-deaza-dGTP, 80 µM dATP, 80 µM dCTP, 80 µM dTTP, 8 µM ddGTP, 50 mM NaCl), an A solution (80 µM 7-deaza-dGTP, 80 µM dATP, 80 µM dCTP, 80 µM dTTP, 8 µM ddATP, 50 mM NaCl), a C solution (80 µM 7-deaza-dGTP, 80 µM dATP, 80 µM dCTP, 80 µM dTTP, 8 µM ddCTP, 50 mM NaCl) and a T solution (80 µM 7-deaza-dGTP, 80 µM dATP, 80 µM dCTP, 80 µM dTTP, 8 µM ddTTP, 50 mM NaCl). In this instance, each of these solutions was kept at 37°C prior to its use. After 5 minutes of reaction at 37°C, the reaction was terminated by adding 4 µl of a reaction termination solution (95% formamide, 0.05% Bromophenol Blue, 20 mM EDTA, 0.05% Xylene Cyanol FF). Thereafter, the reaction mixture was heated at 90°C for 5 minutes, followed by rapid cooling, and a 2.5 µl portion of the resulting sample was subjected to electrophoresis. In this case, a composition consisting of 7 M urea, 10% HydroLink™ LONGRANGER (AT Biochem), 100 mM Tris-HCl, 100 mM boreic acid and 2 mM EDTA was made into gel using 0.05% ammonium persulfate and 0.0005% N,N,N',N'-tetramethylenediamine (TEMED), and the electrophoresis was carried out at a constant power of 60 W using a TEB buffer (50 mM Tris, 50 mM boreic acid, 1 mM EDTA). After completion of the electrophoresis, the gel was dried on a filter paper and subjected to autoradiography to determine the nucleotide sequence of the DNA of interest.

The thus determined DNA sequence is shown in the Sequence ID No. 5, and an amino acid sequence deduced from the DNA sequence is shown in the sequence ID No. 4.

As generally known in this art, the amino acid sequence shown in the Sequence ID No. 4 has a signal peptide. Therefore, the protein of the present invention may be the whole Sequence ID No. 4, a portion of the sequence (for example, the Sequence ID No. 4 except the sequence of a signal peptide), or the portion of the Sequence together with a linker.

The protein of the present invention includes at least an active portion having an activity to enhance the growth of vascular endothelial cells obtainable from a nucleotide sequence or a portion of the nucleotide sequence represented by the Sequence ID No. 5. The DNA corresponding to the signal peptide in the nucleotide sequence represented by the Sequence ID No. 5 may be changed another DNA corresponding to another signal peptide, if necessary, a signal peptide together with a linker DNA sequence may be used in the DNA fragment represented by the Sequence ID No. 5 attached hereto.

### Example 2 Affinity for concanavalin A

The highly purified product obtained in the step (4) of Example 1 was checked for its affinity for concanavalin A in accordance with the procedure described in the foregoing. As the results, it was confirmed that the purified product was possessed of the affinity for concanavalin A, which is a

In addition, on the basis of the results obtained in Examples 1 and 2, it was confirmed that the high purity product of the step (4) was a single chain glycoprotein.

### Example 3 New formation of blood vessels

A total of 10 avian eggs, fertilized for 8 days, were used in each test group. A filter (6 mm in diameter) which has been impregnated with a varied amount of the highly purified product (glycoprotein of this invention) obtained in the step (4) of Example 1 was put on the chorio-allantoic membrane of each egg. After 3 days of incubation at 37°C under a moist condition, new formation of blood vessels was observed under a stereoscopic microscope. The judgement was made as positivre (+, new formation of blood vessels around the filter) or negative (-, no formation of new blood vessels), and the number of positive eggs in each test group was counted. As a comparative example, the same experiment was carried out except that the filter was impregnated with physiological saline instead of the purified product. The results are shown in Table 1.

**Table 1**

| Test group | Amount of glycoprotein | Positive effs/Total |
|---|---|---|
| 1 | 0 (physiological saline) | 0/10 |
| 2 | 1 ng/filter | 1/10 |
| 3 | 10 ng/filter | 3/10 |
| 4 | 50 ng/filter | 5/10 |
| 5 | 100 ng/filter | 6/10 |

It is evident from the above table that the glycoprotein of the present invention is possessed of a function to enhance new formation of blood vessels.

### Example 4 Growth enhancing effect on human umbilical cord vascular endothelial cells

Human umbilical cord vascular endothelial cells were prepared in the usual way and inoculated into a collagen-coated 24 well multi-dish (Corning Glassworks) with a cell density of 1 x 10⁴ cells/well, using MCDB107 medium (Kyokuto Pharmaceutical Industrial Co., Ltd.) supplemented with 20% fetal calf serum. At intervals of 2 days from the next day, the medium was exchanged for a fresh medium containing 5% fetal calf serum and a predetermined amount (see Table 2) of the glycoprotein of the present invention obtained in the step (4) of Example 1. The number of cells was counted on the eighth day, with the results shown in Table 2.

**Table 2**

| Glycoprotein (ng/ml) | Cell count (cells/well) |
|---|---|
| 0 | 27168 |
| 0.3 | 29460 |
| 1.0 | 30920 |
| 3.3 | 37492 |
| 10.0 | 43072 |
| 33.3 | 54772 |
| 100.0 | 53988 |
| 333 | 46460 |

As is evident from the above table, the glycoprotein of the present invention is possessed of a function to enhance the growth of human umbilical cord vascular endothelial cells.

### Example 5 Presence/absence examination of growth enhancing effect on fibroblasts

A primary culture of human dermis fibroblasts prepared from human skin was subcultured, and the eighth subculture was inoculated into a 24 well multi-dish with a cell density of 5 x 10³ cells/well, using DME medium (Flow Laboratories, Inc.) supplemented with 10% fetal calf serum. At intervals of 2 days from the next day, the medium was exchanged for fresh DME medium containing 0.5% fetal calf serum and 100 ng/ml of the glycoprotein of the present invention obtained in the step (4) of Example 1.

As a comparative example, the same procedure was repeated except that the glycoprotein was eliminated from the medium or a basic fibroblast growth factor (bFGF) was used in an amount of 1 ng/ml instead of the glycoprotein.

The number of cells was counted on the eighth day, with the results shown in Table 3.

**Table 3**

| Component added | Cell count on 8th day (cells/well) |
|---|---|
| No addition | 28248 |
| Glycoprotein of Example 1 | 24325 |
| bFGF | 42645 |

As is evident from the above table, bFGF strongly enhances the growth of fibroblasts, but the number of fibroblasts on the eighth day in the case of the addition of the glycoprotein of the present invention obtained in Example 1 is almost the same as that of the case of the control (no addition), thus showing that the inventive glycoprotein hardly has a function to enhance the growth of fibroblasts.

### Example 6 Presence/absence examination of growth enhancing effect on vascular smooth muscle cells

A primary culture of human smooth muscle cells prepared from an umbilical cord was subcultured, and the sixth subculture was inoculated into a 24 well multi-dish with a cell density of 5 x 10³ cells/well, using DME medium supplemented with 10% fetal calf serum. At intervals of 2 days from the next day, the medium was exchanged for fresh medium containing 100 ng/ml of the glycoprotein of the present invention obtained in the step (4) of Example 1.

As a comparative example, the same procedure was repeated except that the glycoprotein was eliminated from the medium or a basic fibroblast growth factor (bFGF) was used in an amount of 1 ng/ml instead of the glycoprotein.

The number of cells was counted on the eighth day, with the results shown in Table 4.

**Table 4**

| Component added | Cell count on 8th day (cells/well) |
|---|---|
| No addition | 6192 |
| Glycoprotein of Example 1 | 7480 |
| bFGF | 48962 |

As is evident from the above table, the number of smooth muscle cells on the eighth day in the case of the addition of the glycoprotein of the present invention obtained in Example 1 is almost the same as that of the case of the control (no addition), thus showing that the inventive glycoprotein has no activity to enhance the growth of human smooth muscle cells.

### Example 7 Presence/absence examination of growth enhancing effect on hepatocytes

Hepatic parenchymal cells (to be referred to as "hepatocytes" hereinafter) were prepared in accordance with the procedure of Takahashi et *al*. (*Tissue Culture*, vol.12, No.8, pp.308 - 312, 1986). The thus prepared hepatocytes were suspended in an inoculation medium (WE basal medium supplemented with 5% fetal calf serum and 10⁻⁸ M dexamethasone) to a cell density of 5.0 x 10⁴ cells/0.2 ml, and the resulting hepatocyte suspension was inoculated into a collagen-coated 24 well multi-dish. After 4 hours of the culturing, the medium was replaced by WE basal medium and the glycoprotein of the present invention obtained in Example 1 was added to the fresh medium in a predetermined amount as shown in Table 5. The same process was repeated after additional 16 hours of the culturing. The medium was exchanged again for fresh WE basal medium 40 hours after the commencement of the culturing, and ³H-thymidine was added to the fresh medium to carry out 2 hours of pulse-labeling. After completion of the pulse-labeling, the culture supernatant was removed, and the remaining cells were washed with a cold phosphate buffer (PBS), 2% perchlorate and 95% cold ethanol in that order and then dried at room temperature. In this instance, each washing step was repeated three times. The thus dried cells in each well were lysed by adding 0.8 ml of a 1% SDS/0.1 N NaOH solution and maintaining the mixture at 37°C for at least 1 hour. A 0.5 ml portion of the resulting lysate was pipetted off from each well and put into a scintillation vial. Thereafter, the content in the vial was mixed with 7 ml of a scintillator (OptiFlow, Packard), and the radioactivity was measured using a scintillation counter to examine ³H-thymidine uptake.

As a comparative example, the same experiment was carried out except that a mixture of insulin (100 nM/ml) and epidermal growth factor (EGF, 50 ng/ml) was used instead of the glycoprotein of the present invention.

The results are shown in Table 5.

**Table 5**

| Component added | Uptake of ³H-thymide |
|---|---|
| Glycoprotein of Example 1 | |
| 300 ng/ml | 5697 DPM |
| 100 ng/ml | 4347 DPM |
| 30 ng/ml | 4869 DPM |
| 10 ng/ml | 4619 DPM |
| Insulin + EGF | 76815 DPM |
| (100 nM + 50 ng/ml) | |
| Control (no addition) | 4992 DPM |

As is evident from the above table, uptake of ³H-thymidine does not occur by the addition of the glycoprotein of the present invention, thus showing that the inventive glycoprotein has no activity to enhance the growth of hepatocytes.

### Example 8 Presence/absence examination of growth enhancing or inhibiting effect on HeLa cells

HeLa-S3 cells were suspended in MEM medium containing 5% bovine serum to a cell density of 1 x 10⁵ cells/ml. The thus prepared HeLa-S3 cell suspension was dispensed in 100 µl portions into wells of a 96 well multi-dish. After 24 hours of culturing, the resulting medium was replaced by fresh MEM medium which has been supplemented writh 5% fetal calf serum and a predetermined amount of the glycoprotein obtained in Example 1, and the culturing was continued for additional 48 hours.

Since the presence or absence of the growth inhibiting effect was not able to be judged clearly with the naked eye under a phase-contrast microscope, the judgement was made by staining the cells with Crystal Violet. That is, each well of the dish after the culturing was washed with a phosphate buffer and then filled with a 10% formalin solution for a period of 30 minutes to fix the cells. The thus treated dish was dried after washing it with running water to remove formalin, and the cells in the dish were stained for 15 minutes with a 0.2% Crystal Violet solution containing 2% ethanol. After removing unbound pigment by washing the dish in running water, and subsequently drying the dish, a predetermined amount of 1% sodium dodecyl sulfate solution was added to each well to dissolve the bound pigment. Thereafter, absorbance of the thus dissolved Crystal Violet was measured at a wave length of 540 nm.

As a control, the same culturing step was repeated except that the glycoprotein was not used, and the Crystal Violet staining and absorbance measurement at 540 nm were carried out in the same manner.

The results are shown in Table 6 in which the absorbance of the control at 540 nm is expressed as 1.00.

**Table 6**

| Component added | Ratio of absorbance at 540 nm |
|---|---|
| Glycoprotein of Example 1 | |
| 300 ng/ml | 1.02 |
| 100 ng/ml | 1.01 |
| 30 ng/ml | 1.01 |
| 10 ng/ml | 1.02 |
| Control (no addition) | 1.00 |

As shown in the above table, the absorbance at 540 nm hardly changed by the addition of the glycoprotein of the present invention in comparison with the case of the control (no addition), thus confirming that the inventive glycoprotein has no activity to enhance or inhibit the growth of HeLa cells.

### Example 9 Migration-stimulating activity on vascular endothelial cells and smooth muscle cells

Primary culturing of vascular endothelial cells was carried out by isolating the cells from rabbit cornea capillary vessels in the usual way. The migration-stimulating activity of the cells was measured in accordance with the Boyden's test using Boyden's chamber. That is, DME medium supplemented with 10% fetal calf serum and a predetermined amount of the glycoprotein obtained in Example 1 was put into the lower compartment of the Boyden's chamber, and another DME medium supplemented with 10% fetal calf serum and 2 x 10⁴/ml of vascular endothelial cells was put into the upper compartment of the chamber. Thereafter, culturing was carried out at 37°C for 4 hours.

A similar test was carried out using primary-cultured smooth muscle cells which have been isolated from rat pulmonary artery

After the culturing, the thus treated cells were stained with Diff-Quick solution, and the number of migrated cells per visual field was counted under a microscope, with the results shown in Table 7.

**Table 7**

| | The number of migrated cells | |
|---|---|---|
| Glycoprotein | Vascular endothelial cells | Smooth muscle cells |
| 300 ng/ml | 268 | 0 |
| 100 ng/ml | 50 | 0 |
| 30 ng/ml | 37 | 0 |

As is evident from the above table, the glycoprotein of the present invention shows migration-stimulating activity on vascular endothelial cells but not on smooth muscle cells.

Thus, it is apparent that there has been provided, in accordance with the present invention, a novel protein of human origin, as well as a process for the production thereof. Since the protein of the present invention enhances the growth of vascular endothelial cells but does not activate the growth of smooth muscle cells, fibroblasts and hepatocytes and also does not enhance or inhibit the growth of HeLa cells, it can enhance the growth of vascular endothelial cells selectively and therefore can enhance new formation of blood vessels smoothly without causing secondary reactions. Because of such excellent properties, especially its activity to enhance new formation of blood vessels, the protein of the present invention can be applied to a healing enhancer of wound, burn injury, decubitus, postoperative tissue damage or the like or as a drug for the treatment of cardiac angiopathy, as well as its application to artificial organs such as artificial blood vessel, artificial skin and the like. It also can be applied to diagnostic and therapeutic drugs of malignant tumor, retinopathy, chronic rheumatoid arthritis and the like.

In addition, the protein of the present invention can be obtained with a high productivity and a high purity in comparison with the prior art physiologically active factors.

## Claims

1. A single chain protein selectively enhancing the growth of vascular endothelial cells, characterized in that it comprises the following peptide chains : 
(SEQ. ID No. : 1)
Arg Asn Thr Ile His Glu Phe
 1               5
(SEQ. ID No. : 2)
Glu Phe Gly His Glu Phe Asp Leu Tyr Glu
 1               5                   10
(SEQ. ID No. : 3)
Glu Ser Xaa Val Leu Thr Ala Arg Gln Xaa Phe Pro Ser Arg Asp Leu
 1               5                   10                  15
 and in that it has a molecular weight of from 72,000 to 80,000 Da when determined by SDS polyacrylamide gel electrophoresis or from 79,000 to 85,000 Da when determined under reducing conditions.

2. A process for producing the protein according to claim 1 which comprises purifying a serum-free culture supernatant of said human ovarian tumor established cell line, HUOCA-II or HUOCA-III, by combining purification techniques including (a) cation exchange chromatography, (b) heparin affinity chromatography, (c) heparin affinity high performance liquid chromatography and (d) reverse phase high performance liquid chromatography.

3. A protein of human origin which contains an amino acid sequence or a portion of the amino acid sequence represented by the following sequence (SEQ ID No. : 4) :

Met Trp Val Thr Lys Leu Leu Pro Ala Leu Leu Leu Gln His Val
1                                   10
Leu Leu His Leu Leu Leu Leu Pro Ile Ala Ile Pro Tyr Ala Glu
                20                                      30
Gly Gln Arg Lys Arg Arg Asn Thr Ile His Glu Phe Lys Lys Ser
                                    40
Ala Lys Thr Thr Leu Ile Lys Ile Asp Pro Ala Leu Lys Ile Lys
                50                                      60

Thr Lys Lys Val Asn Thr Ala Asp Gln Cys Ala Asn Arg Cys Thr
                                        70
Arg Asn Lys Gly Leu Pro Phe Thr Cys Lys Ala Phe Val Phe Asp
                80                                      90
Lys Ala Arg Lys Gln Cys Leu Trp Phe Pro Phe Asn Ser Met Ser
                                        100
Ser Gly Val Lys Lys Glu Phe Gly His Glu Phe Asp Leu Tyr Glu
                110                                     120
Asn Lys Asp Tyr Ile Arg Asn Cys Ile Ile Gly Lys Gly Arg Ser
                                        130
Tyr Lys Gly Thr Val Ser Ile Thr Lys Ser Gly Ile Lys Cys Gln
                140                                     150
Pro Trp Ser Ser Met Ile Pro His Glu His Ser Phe Leu Pro Ser
                                        160
Ser Tyr Arg Gly Lys Asp Leu Gln Glu Asn Tyr Cys Arg Asn Pro
                170                                     180
Arg Gly Glu Glu Gly Gly Pro Trp Cys Phe Thr Ser Asn Pro Glu
                                        190
Val Arg Tyr Glu Val Cys Asp Ile Pro Gln Cys Ser Glu Val Glu
                200                                     210
Cys Met Thr Cys Asn Gly Glu Ser Tyr Arg Gly Leu Met Asp His
                                        220
Thr Glu Ser Gly Lys Ile Cys Gln Arg Trp Asp His Gln Thr Pro
                230                                     240
His Arg His Lys Phe Leu Pro Glu Arg Tyr Pro Asp Lys Gly Phe
                                        250
Asp Asp Asn Tyr Cys Arg Asn Pro Asp Gly Gln Pro Arg Pro Trp
                260                                     270
Cys Tyr Thr Leu Asp Pro His Thr Arg Trp Glu Tyr Cys Ala Ile
                                        280
Lys Thr Cys Ala Asp Asn Thr Met Asn Asp Thr Asp Val Pro Leu
                290                                     300
Glu Thr Thr Glu Cys Ile Gln Gly Gln Gly Glu Gly Tyr Arg Gly
                                        310
Thr Val Asn Thr Ile Trp Asn Gly Ile Pro Cys Gln Arg Trp Asp
                320                                     330
Ser Gln Tyr Pro His Glu His Asp Met Thr Pro Glu Asn Phe Lys
                                        340
Cys Lys Asp Leu Arg Glu Asn Tyr Cys Arg Asn Pro Asp Gly Ser
                350                                     360
Glu Ser Pro Trp Cys Phe Thr Thr Asp Pro Asn Ile Arg Val Gly
                                        370
Tyr Cys Ser Gln Ile Pro Asn Cys Asp Met Ser His Gly Gln Asp
                380                                     390
Cys Tyr Arg Gly Asn Gly Lys Asn Tyr Met Gly Asn Leu Ser Gln
                                        400
Thr Arg Ser Gly Leu Thr Cys Ser Met Trp Asp Lys Asn Met Glu
                410                                     420
Asp Leu His Arg His Ile Phe Trp Glu Pro Asp Ala Ser Lys Leu
                                        430
Asn Glu Asn Tyr Cys Arg Asn Pro Asp Asp Asp Ala His Gly Pro
                440                                     450
Trp Cys Tyr Thr Gly Asn Pro Leu Ile Pro Trp Asp Tyr Cys Pro
                                        460

Ile Ser Arg Cys Glu Gly Asp Thr Thr Pro Thr Ile Val Asn Leu
470 480
Asp His Pro Val Ile Ser Cys Ala Lys Thr Lys Gln Leu Arg Val
490
Val Asn Gly Ile Pro Thr Arg Thr Asn Ile Gly Trp Met Val Ser
500 510
Leu Arg Tyr Arg Asn Lys His Ile Cys Gly Gly Ser Leu Ile Lys
520
Glu Ser Trp Val Leu Thr Ala Arg Gln Cys Phe Pro Ser Arg Asp
530 540
Leu Lys Asp Tyr Glu Ala Trp Leu Gly Ile His Asp Val His Gly
550
Arg Gly Asp Glu Lys Cys Lys Gln Val Leu Asn Val Ser Gln Leu
560 570
Val Tyr Gly Pro Glu Gly Ser Asp Leu Val Leu Met Lys Leu Ala
580
Arg Pro Ala Val Leu Asp Asp Phe Val Ser Thr Ile Asp Leu Pro
590 600
Asn Tyr Gly Cys Thr Ile Pro Glu Lys Thr Ser Cys Ser Val Tyr
610
Gly Trp Gly Tyr Thr Gly Leu Ile Asn Tyr Asp Gly Leu Leu Arg
620 630
Val Ala His Leu Tyr Ile Met Gly Asn Glu Lys Cys Ser Gln His
640
His Arg Gly Lys Val Thr Leu Asn Glu Ser Glu Ile Cys Ala Gly
650 660
Ala Glu Lys Ile Gly Ser Gly Pro Cys Glu Gly Asp Tyr Gly Gly
670
Pro Leu Val Cys Glu Gln His Lys Met Arg Met Val Leu Gly Val
680 690
Ile Val Pro Gly Arg Gly Cys Ala Ile Pro Asn Arg Pro Gly Ile
700
Phe Val Arg Val Ala Tyr Tyr Ala Lys Trp Ile His Lys Ile Ile
710 720
Leu Thr Tyr Lys Val Pro Gln Ser 728

4. A pharmaceutical composition which contains the protein of claim 1 or 3 as an active ingredient.

5. A DNA fragment which contains a nucleotide sequence or a portion of the nucleotide sequence below (SEQ ID No. : 5) : 
ATG TGG GTG ACC AAA CTC CTG CCA GCC CTG CTG CTG CAG CAT
1
GTC CTC CTG CAT CTC CTC CTG CTC CCC ATC GCC ATC CCC TAT
45
GCA GAG GGA CAA AGG AAA AGA AGA AAT ACA ATT CAT GAA TTC
93
AAA AAA TCA GCA AAG ACT ACC CTA ATC AAA ATA GAT CCA GCA
141
CTG AAG ATA AAA ACC AAA AAA GTG AAT ACT GCA GAC CAA TGT
189
GCT AAT AGA TGT ACT AGG AAT AAA GGA CTT CCA TTC ACT TGC
237
 
AAG GCT TTT GTT TTT GAT AAA GCA AGA AAA CAA TGC CTC TGG
285
TTC CCC TTC AAT AGC ATG TCA AGT GGA GTG AAA AAA GAA TTT
333
GGC CAT GAA TTT GAC CTC TAT GAA AAC AAA GAC TAC ATT AGA
AAC TGC ATC ATT GGT AAA GGA CGC AGC TAC AAG GGA ACA GTA
381
TCT ATC ACT AAG AGT GGC ATC AAA TGT CAG CCC TGG AGT TCC
429
ATG ATA CCA CAC GAA CAC AGC TTT TTG CCT TCG AGC TAT CGG
477
GGT AAA GAC CTA CAG GAA AAC TAC TGT CGA AAT CCT CGA GGG
525
GAA GAA GGG GGA CCC TGG TGT TTC ACA AGC AAT CCA GAG GTA
573
CGC TAC GAA GTC TGT GAC ATT CCT CAG TGT TCA GAA GTT GAA
621
TGC ATG ACC TGC AAT GGG GAG AGT TAT CGA GGT CTC ATG GAT
699
CAT ACA GAA TCA GGC AAG ATT TGT CAG CGC TGG GAT CAT CAG
ACA CCA CAC CGG CAC AAA TTC TTG CCT GAA AGA TAT CCC GAC
717
AAG GGC TTT GAT GAT AAT TAT TGC CGC AAT CCC GAT GGC CAG
765
CCG AGG CCA TGG TGC TAT ACT CTT GAC CCT CAC ACC CGC TGG
813
GAG TAC TGT GCA ATT AAA ACA TGC GCT GAC AAT ACT ATG AAT
861
GAC ACT GAT GTT CCT TTG GAA ACA ACT GAA TGC ATC CAA GGT
909
CAA GGA GAA GGC TAC AGG GGC ACT GTC AAT ACC ATT TGG AAT
957
GGA ATT CCA TGT CAG CGT TGG GAT TCT CAG TAT CCT CAC GAG
1005
CAT GAC ATG ACT CCT GAA AAT TTC AAG TGC AAG GAC CTA CGA
GAA AAT TAC TGC CGA AAT CCA GAT GGG TCT GAA TCA CCC TGG
1053
TGT TTT ACC ACT GAT CCA AAC ATC CGA GTT GGC TAC TGC TCC
1101
CAA ATT CCA AAC TGT GAT ATG TCA CAT GGA CAA GAT TGT TAT
1149
CGT GGG AAT GGC AAA AAT TAT ATG GGC AAC TTA TCC CAA ACA
1197
AGA TCT GGA CTA ACA TGT TCA ATG TGG GAC AAG AAC ATG GAA
1245
GAC TTA CAT CGT CAT ATC TTC TGG GAA CCA GAT GCA AGT AAG
1293
CTG AAT GAG AAT TAC TGC CGA AAT CCA GAT GAT GAT GCT CAT
1341
GGA CCC TGG TGC TAC ACG GGA AAT CCA CTC ATT CCT TGG GAT
TAT TGC CCT ATT TCT CGT TGT GAA GGT GAT ACC ACA CCT ACA
1389
ATA GTC AAT TTA GAC CAT CCC GTA ATA TCT TGT GCC AAA ACG
1437
AAA CAA TTG CGA GTT GTA AAT GGG ATT CCA ACA CGA ACA AAC
1485
 
ATA GGA TGG ATG GTT AGT TTG AGA TAC AGA AAT AAA CAT ATC
1533
TGC GGA GGA TCA TTG ATA AAG GAG AGT TGG GTT CTT ACT GCA
1581
CGA CAG TGT TTC CCT TCT CGA CAC TTG AAA GAT TAT GAA GCT
1629
TGG CTT GGA ATT CAT GAT GTC CAC GGA AGA GGA GAT GAG AAA
1677
TGC AAA CAG GTT CTC AAT GTT TCC CAG CTG GTA TAT GGC CCT

GAA GGA TCA GAT CTG GTT TTA ATG AAG CTT GCC AGG CCT GCT
1725
GTC CTG GAT GAT TTT GTT AGT ACG ATT GAT TTA CCT AAT TAT
1773

GGA TGC ACA ATT CCT GAA AAG ACC AGT TGC AGT GTT TAT GGC
1821
TGG GGC TAC ACT GGA TTG ATC AAC TAT GAT GGC CTA TTA CGA
1869
GTG GCA CAT CTC TAT ATA ATG GGA AAT GAG AAA TGC AGC CAG
1917
CAT CAT CGA GGG AAG GTG ACT CTG AAT GAG TCT GAA ATA TGT
1965
GCT GGG GCT GAA AAG ATT GGA TCA GGA CCA TGT GAG GGG GAT
2013
TAT GGT GGC CCA CTT GTT TGT GAG CAA CAT AAA ATG AGA ATG

GTT CTT GGT GTC ATT GTT CCT GGT CGT GGA TGT GCC ATT CCA
2061
AAT CGT CCT GGT ATT TTT GTC CGA GTA GCA TAT TAT GCA AAA
2109
TGG ATA CAC AAA ATT ATT TTA ACA TAT AAG GTA CCA CAG TCA
2157
TAG 2187
 wherein at least one base may be substituted based on the degeneracy of genetic code.

6. A single chain protein having an activity to enhance the growth of vascular endothelial cells obtainable from the DNA fragment of claim 5.

7. A DNA fragment complementary to the DNA fragment of claim 5.

8. An expression vector which contains the DNA fragment of claim 5.

9. A transformant transformed with the DNA fragment of claim 5.

10. A transformant transformed with the expression vector of claim 8.
